# EUROPEAN PATENT APPLICATION

(11) **EP 4 725 964 A1**
(43) Date of publication of application: **15.04.2026**
(21) Application number: 24818787.4
(22) Date of filing: 07.06.2024
(51) Int. Cl.: C07K 16/28, C07K 16/30, C12N 15/63, C12N 5/10, G01N 33/53, G01N 33/574, G01N 33/577, A61K 39/00

(54) **ANTIBODY BINDING TO NECTIN-4 AND USE THEREOF**

(30) Priority: 08.06.2023 CN 202310675844
(71) Applicant: Jiangsu Mabwell Health Pharmaceutical R&D Co., Ltd., China Medical City Taizhou, Jiangsu 225300 (CN); Mabwell (Shanghai) Bioscience Co., Ltd., Shanghai 201210 (CN)
(72) Inventor: FANG, Peng, Taizhou, Jiangsu 225300 (CN); TAN, Xiaoding, Taizhou, Jiangsu 225300 (CN); YOU, Meng, Taizhou, Jiangsu 225300 (CN); CAO, Yuxia, Taizhou, Jiangsu 225300 (CN); WANG, Lu, Taizhou, Jiangsu 225300 (CN); XI, Zhao, Taizhou, Jiangsu 225300 (CN); SHI, Lei, Taizhou, Jiangsu 225300 (CN); ZHU, Xiaohong, Taizhou, Jiangsu 225300 (CN)
(74) Representative: Bianchetti & Minoja with Trevisan & Cuonzo IPS SRL
(86) International application number: PCT/CN2024/098089
(87) International publication number: WO 2024/251260

(57) **Abstract**

Provided are an antibody and an antigen-binding fragment that specifically bind to Nectin-4, and a test kit containing the antibody or the antigen-binding fragment. Also provided are a nucleic acid encoding the antibody, a host cell containing the nucleic acid, and a method for preparing the antibody. Also provided are uses of the antibody specifically binding to Nectin-4 in diagnosis and prognosis.

## Description

### Technical Field

The present invention relates to the field of antibody medicine. Particularly, the present invention relates to an antibody or an antigen-binding fragment thereof that specifically bind to Nectin-4, as well as a kit comprising the antibody or the antigen-binding fragment. Further, the present invention relates to a nucleic acid encoding the antibody, a host cell comprising the nucleic acid, and a method for preparing the antibody. The present invention also relates to uses of the antibody binding to Nectin-4 in diagnosis and prognosis.

### Background Art

Nectin-4 (also known as "Poliovirus Receptor-Like Molecule 4 (PVRL4)") is a type I transmembrane glycoprotein with a molecular weight of approximately 66 KD. It belongs to a member of Nectin family within the immunoglobulin superfamily. Its extracellular domain consists of three Ig like domains (VCC type), and it cooperates with cadherins to participate in the formation and maintenance of adherens junctions.

Nectin-4 is specifically expressed in embryos, placentas, and tumor cells, while showing no or extremely low expression in normal tissues and organs. It serves as a classic clinical tumor marker and an ideal target for an antibody-drug conjugate (ADC). ADC targeting Nectin-4 binds to the overexpressed Nectin-4 on the surface of tumor cells, forming an ADC-receptor complex. The ADC subsequently undergoes target-mediated endocytosis, allowing the ADC to enter the cells. Within lysosomes, the ADC is cleaved at the linker therein by cathepsins, releasing toxic small molecules to achieve specific killing of tumor cells. PADCEV^{™} (also known as enfortumab vedotin-ejfv; Chinese name: enfortumab vedotin) is an ADC drug, which is formed by conjugating a human IgG1 monoclonal antibody, enfortumab targeting the Nectin-4 protein, with a cytotoxic agent MMAE (monomethyl auristatin E, a microtubule disruptor) (Pia M. Challita-Eid et al. (2016) Cancer Res.76(10):3003-13). It is FDA-approved for treating locally advanced or metastatic urothelial carcinoma. However, PADCEV^{™} targeting Nectin-4 is intended solely for therapeutic use and cannot be employed for immunohistochemical (IHC) staining, thus lacking diagnostic utility.

There exists a need in the field for an anti-Nectin-4 antibody that specifically recognizes Nectin-4 and can highly sensitively assay the presence and/or expression levels of Nectin-4 in a sample (e.g., via IHC assay), so as to achieve a disease diagnosis or prognosis assessment, which is of significant importance for improving cancer patients' survival rate, prolonging survival, avoiding overtreatment with chemotherapy, and enhancing quality of life. Therefore, the use of a highly specific and sensitive anti-Nectin-4 antibody in diagnosis and prognosis holds immense practical value.

### Summary of the Invention

The present invention provides an anti-Nectin-4 antibody with high specificity and sensitivity, capable of reliably and sensitively detecting Nectin-4. The antibody or the antigen-binding fragment of the present invention that specifically binds to Nectin-4 has one or more of the following properties:
(a) possessing the ability to bind specifically only to Nectin-4, with no specific binding to Nectin family members of Nectin-1, Nectin-2, Nectin-3, Nectin-like-1, Nectin-like-2, Nectin-like-3, Nectin-like-4, and Nectin-like-5, as measured in an ELISA assay;
(b) possessing the ability to bind to Nectin-4 molecule expressed on cells, as measured in a flow cytometry assay;
(c) capable of specifically detect Nectin-4 protein; and the binding to Nectin-4 protein on the membrane is inhibited when excess Nectin-4 protein is added, as measured in Western blotting; and/or
(d) specifically staining tissues expressing Nectin-4 molecule, as measured in immunohistochemical staining, for example, the tissues are selected from a group consisting of tonsil, salivary gland, brain, cerebellum, placenta, bladder, lung, mammary gland, esophagus, larynx, thymus, heart, stomach, small intestine, colon, rectum, ureter, ovary, fallopian tube, cervix, endometrium, skin, kidney, prostate, pancreas, thyroid, spleen, and liver.

Therefore, in a first aspect, the present invention provides an antibody or an antigen-binding fragment that specifically binds to Nectin-4, comprising a heavy chain variable region and a light chain variable region, wherein:
(a) the heavy chain variable region comprises HCDR1 shown in SEQ ID NO: 5 or a variant of HCDR1 shown in SEQ ID NO: 5 with no more than two amino acid changes, HCDR2 shown in SEQ ID NO: 6 or a variant of HCDR2 shown in SEQ ID NO: 6 with no more than two amino acid changes, and HCDR3 shown in SEQ ID NO: 7 or a variant of HCDR3 shown in SEQ ID NO: 7 with no more than two amino acid changes; the light chain variable region comprises LCDR1 shown in SEQ ID NO: 8 or a variant of LCDR1 shown in SEQ ID NO: 8 with no more than two amino acid changes, LCDR2 shown in SEQ ID NO: 9 or a variant of LCDR2 shown in SEQ ID NO: 9 with no more than two amino acid changes, and LCDR3 shown in SEQ ID NO: 10 or a variant of LCDR3 shown in SEQ ID NO: 10 with no more than two amino acid changes;
(b) the heavy chain variable region comprises HCDR1 shown in SEQ ID NO: 15 or a variant of HCDR1 shown in SEQ ID NO: 15 with no more than two amino acid changes, HCDR2 shown in SEQ ID NO: 16 or a variant of HCDR2 shown in SEQ ID NO: 16 with no more than two amino acid changes, and HCDR3 shown in SEQ ID NO: 17 or a variant of HCDR3 shown in SEQ ID NO: 17 with no more than two amino acid changes; the light chain variable region comprises LCDR1 shown in SEQ ID NO: 18 or a variant of LCDR1 shown in SEQ ID NO: 18 with no more than two amino acid changes, LCDR2 shown in SEQ ID NO: 19 or a variant of LCDR2 shown in SEQ ID NO: 19 with no more than two amino acid changes, and LCDR3 shown in SEQ ID NO: 20 or a variant of LCDR3 shown in SEQ ID NO: 20 with no more than two amino acid changes; or
(c) the heavy chain variable region comprises HCDR1 shown in SEQ ID NO: 25 or a variant of HCDR1 shown in SEQ ID NO: 25 with no more than two amino acid changes, HCDR2 shown in SEQ ID NO: 26 or a variant of HCDR2 shown in SEQ ID NO: 26 with no more than two amino acid changes, and HCDR3 shown in SEQ ID NO: 27 or a variant of HCDR3 shown in SEQ ID NO: 27 with no more than two amino acid changes; the light chain variable region comprises LCDR1 shown in SEQ ID NO: 28 or a variant of LCDR1 shown in SEQ ID NO: 28 with no more than two amino acid changes, LCDR2 shown in SEQ ID NO: 29 or a variant of LCDR2 shown in SEQ ID NO: 29 with no more than two amino acid changes, and LCDR3 shown in SEQ ID NO: 30 or a variant of LCDR3 shown in SEQ ID NO: 30 with no more than two amino acid changes.

In some embodiments, the amino acid changes are conservative amino acid modifications.

In some embodiments, the antibody or the antigen-binding fragment that specifically binds to Nectin-4 of the present invention comprises
(a) a heavy chain variable region and a light chain variable region, wherein the heavy chain variable region comprises a sequence of SEQ ID NO: 3 or a sequence having at least 90%, 91%, 92%, 93%, 94%, 95%, 96%, 97%, 98%, or 99% identity therewith, and the light chain variable region comprises a sequence of SEQ ID NO: 4 or a sequence having at least 90%, 91%, 92%, 93%, 94%, 95%, 96%, 97%, 98%, or 99% identity therewith;
(b) a heavy chain variable region and a light chain variable region, wherein the heavy chain variable region comprises a sequence of SEQ ID NO: 13 or a sequence having at least 90%, 91%, 92%, 93%, 94%, 95%, 96%, 97%, 98%, or 99% identity therewith, and the light chain variable region comprises a sequence of SEQ ID NO: 14 or a sequence having at least 90%, 91%, 92%, 93%, 94%, 95%, 96%, 97%, 98%, or 99% identity therewith; or
(c) a heavy chain variable region and a light chain variable region, wherein the heavy chain variable region comprises a sequence of SEQ ID NO: 23 or a sequence having at least 90%, 91%, 92%, 93%, 94%, 95%, 96%, 97%, 98%, or 99% identity therewith, and the light chain variable region comprises a sequence of SEQ ID NO: 24 or a sequence having at least 90%, 91%, 92%, 93%, 94%, 95%, 96%, 97%, 98%, or 99% identity therewith.

In some embodiments, the antigen-binding fragment of the antibody that specifically binds to Nectin-4 of the present invention is Fab, Fab', F(ab')₂, Fv, single-chain Fv, single-chain Fab, or diabody.

In some embodiments, the antibody or the antigen-binding fragment that specifically binds to Nectin-4 of the present invention comprises
(a) a heavy chain sequence of SEQ ID NO: 1 or having at least 90%, 91%, 92%, 93%, 94%, 95%, 96%, 97%, 98%, or 99% identity therewith, and a light chain sequence of SEQ ID NO: 2 or having at least 90%, 91%, 92%, 93%, 94%, 95%, 96%, 97%, 98%, or 99% identity therewith; e.g., the heavy chain sequence shown in SEQ ID NO: 1 and the light chain sequence shown in SEQ ID NO: 2;
(b) a heavy chain sequence of SEQ ID NO: 11 or having at least 90%, 91%, 92%, 93%, 94%, 95%, 96%, 97%, 98%, or 99% identity therewith, and a light chain sequence of SEQ ID NO: 12 or having at least 90%, 91%, 92%, 93%, 94%, 95%, 96%, 97%, 98%, or 99% identity therewith; e.g., the heavy chain sequence shown in SEQ ID NO: 11 and the light chain sequence shown in SEQ ID NO: 12; or
(c) a heavy chain sequence of SEQ ID NO: 21 or having at least 90%, 91%, 92%, 93%, 94%, 95%, 96%, 97%, 98%, or 99% identity therewith, and a light chain sequence of SEQ ID NO: 22 or having at least 90%, 91%, 92%, 93%, 94%, 95%, 96%, 97%, 98%, or 99% identity therewith; e.g., the heavy chain sequence shown in SEQ ID NO: 21 and the light chain sequence shown in SEQ ID NO: 22.

In some embodiments, the antibody or the antigen-binding fragment that specifically binds to Nectin-4 of the present invention is a mouse antibody or a rabbit antibody.

In some embodiments, the antibody or the antigen-binding fragment that specifically binds to Nectin-4 of the present invention is an IgG class antibody.

In a second aspect, the present invention provides a nucleic acid encoding the anti-Nectin-4 antibody or the antigen-binding fragment described in the first aspect above, a vector (preferably an expression vector) comprising the nucleic acid, and a host cell comprising the nucleic acid or the vector. In some embodiments, the host cell is prokaryotic or eukaryotic, for example, selected from a group consisting of E. coli cells, yeast cells, mammalian cells, or other cells suitable for preparing an antibody or an antigen-binding fragment thereof. In some embodiments, the host cell is HEK 293 cell or CHO cell.

In a third aspect, the present invention provides a method for preparing the anti-Nectin-4 antibody or the antigen-binding fragment thereof described in the first aspect above, comprising: culturing the host cell described in the second aspect above under conditions suitable for expressing the nucleic acid encoding the anti-Nectin-4 antibody or the antigen-binding fragment thereof described in the first aspect above, and recovering the expressed anti-Nectin-4 antibody or antigen-binding fragment thereof from the culture.

In a fourth aspect, the present invention provides a kit comprising the anti-Nectin-4 antibody or the antigen-binding fragment described in the first aspect above.

In a fifth aspect, the present invention provides a use of the anti-Nectin-4 antibody or the antigen-binding fragment thereof described in the first aspect above for detecting Nectin-4 level in a sample.

In a sixth aspect, the present invention provides a method for diagnosing cancer in a subject using the anti-Nectin-4 antibody or the antigen-binding fragment thereof described in the first aspect above.

In some embodiments, the present invention provides a method for determining a subject's eligibility for treatment with an anti-Nectin-4 antibody or an anti-Nectin-4 antibody-drug conjugate (e.g., an anti-Nectin-4 antibody or an antigen-binding fragment thereof with therapeutic efficacy), using the anti-Nectin-4 antibody or the antigen-binding fragment thereof described in the first aspect above.

In some embodiments, the present invention provides a method for prognosing a subject with cancer, using the anti-Nectin-4 antibody or the antigen-binding fragment thereof described in the first aspect above, wherein the subject with cancer is one who has been treated with an anti-tumor agent (e.g., an anti-Nectin-4 antibody or an anti-Nectin-4 antibody-drug conjugate (e.g., an anti-Nectin-4 antibody or an antigen-binding fragment thereof with therapeutic efficacy)).

### Brief Description of the Drawings

The preferred embodiments of the present invention described in detail below will be better understood when read in conjunction with the accompanying drawings. For the purpose of illustrating the present invention, the drawings show currently preferred embodiments. However, it should be understood that the present invention is not limited to the precise arrangement and means shown in the drawings.
Figure 1 shows the assay of the binding of antibody MM11, antibody R012, and antibody R036 to Nectin family proteins (Nectin-1, Nectin-2, Nectin-3, Nectin-4, Nectin-like-1, Nectin-like-2, Nectin-like-3, Nectin-like-4, Nectin-like-5) via ELISA.
Figure 2 shows the assay of the binding of antibody MM11, antibody R012, and antibody R036 to Nectin-4-expressing cells via flow cytometry (FACS). In the figure, "Blank" indicates human prostate cancer cells (PC-3) treated only with a secondary antibody, without adding antibody MM11, antibody R012, or antibody R036 as a primary antibody; "NC" indicates PC-3 cells treated only with blank buffer; "PC-3-Nectin4" indicates a PC-3 cell line expressing Nectin-4.

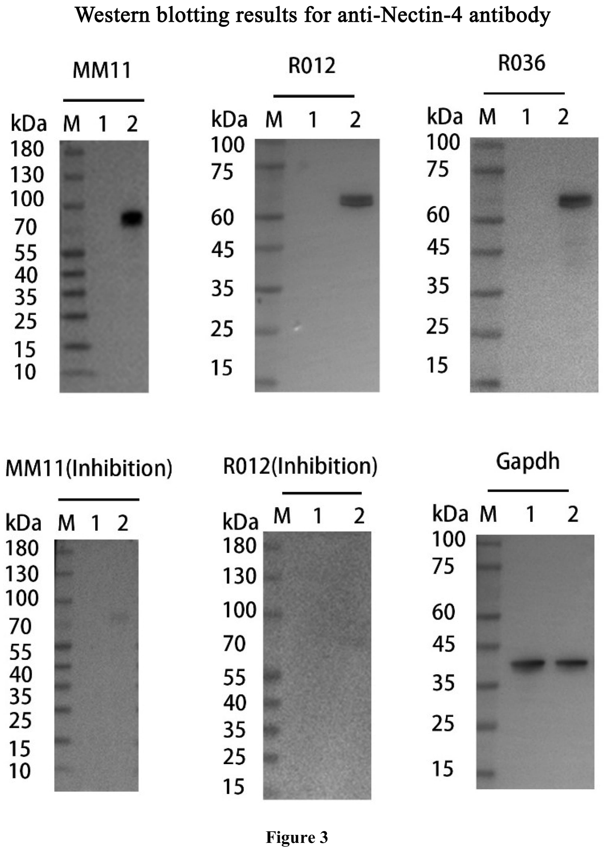

Figure 3 shows the reactivity of antibody MM11, antibody R012, and antibody R036 with Nectin-4-expressing cell lysates as detected by Western blotting. In the figure, Lane M: Protein molecular weight marker; Lane 1: PC-3 cell lysate; Lane 2: PC-3-Nectin-4 cell lysate. The upper panels of Figure 3 show that antibody MM11, antibody R012, and antibody R036 each react with lysates from Nectin-4-expressing cells, but not with lysates from cells that do not express Nectin-4. In the lower panels of Figure 3, "MM11(Inhibition)" and "R012(Inhibition)" indicate the results obtained by incubating membrane-transferred cell lysates with "MM11 antibody" or "R012 antibody" respectively, in the presence of excess recombinant Nectin-4 protein being added; "Gapdh" indicates the reaction of incubating membrane-transferred cell lysates with an anti-glyceraldehyde-3-phosphate dehydrogenase antibody, serving as an internal reference for Western blotting.
Figure 4 shows immunohistochemical staining results of representative normal human tissues using Nectin-4 rabbit monoclonal antibodies R012, R036, and the positive control Nectin-4 rabbit monoclonal antibody EPR15613-68 (scale bar: 200 µm).
Figure 5 shows immunohistochemical staining results of human bladder cancer tissues using Nectin-4 rabbit monoclonal antibodies R012, R036, and the positive control Nectin-4 rabbit monoclonal antibody EPR15613-68 (scale bar: 200 µm).
Figure 6 shows immunohistochemical staining results of human lung cancer tissues using Nectin-4 rabbit monoclonal antibodies R012, R036, and the positive control Nectin-4 rabbit monoclonal antibody EPR15613-68 (scale bar: 200 µm).
Figure 7 shows immunohistochemical staining results of human breast cancer tissues using Nectin-4 rabbit monoclonal antibodies R012, R036, and the positive control Nectin-4 rabbit monoclonal antibody EPR15613-68 (scale bar: 200 µm).
Figure 8 shows immunohistochemical staining results of representative normal human tissues using Nectin-4 mouse monoclonal antibody MM11, and the positive control Nectin-4 mouse monoclonal antibody M22-321b41.1 (scale bar: 100 µm).
Figure 9 shows immunohistochemical staining results of human prostate cancer tissues using Nectin-4 mouse monoclonal antibody MM11, and the positive control Nectin-4 mouse monoclonal antibody M22-321b41.1 (scale bar: 50 µm).
Figure 10 shows immunohistochemical staining results of human lung cancer tissues using Nectin-4 mouse monoclonal antibody MM11, and the positive control Nectin-4 mouse monoclonal antibody M22-321b41.1 (scale bar: 50 µm).
Figure 11 shows immunohistochemical staining results of human triple-negative breast cancer tissues using Nectin-4 mouse monoclonal antibody MM11, and the positive control Nectin-4 mouse monoclonal antibody M22-321b41.1 (scale bar: 50 µm).

### Detailed Description

Before detailing the present invention, it should be understood that the present invention is not limited to the specific methods and experimental conditions described herein, as the methods and conditions may be modified.

### I. Definitions

Unless otherwise specified, all technical and scientific terms used herein shall have the same meanings as commonly understood by a person skilled in the art to which the present invention pertains.

The term "about" used in conjunction with a numerical value is intended to encompass the numerical values in a range from a lower limit less than the specified numerical value by 5% to an upper limit greater than the specified numerical value by 5%.

As used herein, the term "and/or" refers to any one of the options, or any two or more of the options.

The term "comprise" or "include" used herein, unless otherwise specified, also encompasses the scenario where the subject matter consists of the described elements, integers or steps. For example, when referring to an antibody variable region "comprising" a particular sequence, it is also intended to encompass an antibody variable region consisting of the particular sequence.

As used herein, the term "antibody" refers to an immunoglobulin molecule that binds to an antigen. Embodiments of an antibody include a monoclonal antibody, a polyclonal antibody, or a chimeric antibody. An antibody may belong to any class (e.g., IgG, IgE, IgM, IgD, IgA).

The exemplary antibody of the present disclosure is an antibody of immunoglobulin G (IgG) class, consisting of four polypeptide chains, i.e., two heavy chains (HC) and two light chains (LC), cross-linked by interchain disulfide bonds. The amino-terminal portion of each of the four polypeptide chains includes a variable region comprising about 100-125 or more amino acids primarily responsible for antigen recognition. Each heavy chain consists of a heavy chain variable region (VH) and a heavy chain constant region. Each light chain consists of a light chain variable region (VL) and a light chain constant region.

The VH and VL regions can be further subdivided into highly variable regions known as complementarity-determining regions (CDRs), interspersed with more conserved regions known as framework regions (FRs). CDRs are exposed on the surface of the protein and constitute the critical regions responsible for an antibody's antigen-binding specificity. Each VH and VL comprises three CDRs and four FRs arranged in the following order from amino terminus to carboxyl terminus: FR1, CDR1, FR2, CDR2, FR3, CDR3, FR4. The three CDRs of the heavy chain are termed as "HCDR1, HCDR2, and HCDR3", while the three CDRs of the light chain are termed as "LCDR1, LCDR2, and LCDR3". CDRs contain most residues responsible for forming antigen-specific interactions. Amino acid residues can be assigned to CDRs according to well-established numbering schemes, including those described by Kabat (Kabat et al., "Sequences of Proteins of Immunological Interest", National Institutes of Health, Bethesda, Md.(1991)); Chothia (Chothia et al., "Canonical structures for the hypervariable regions of immunoglobulins", Journal of Molecular Biology, 196, 901-917 (1987); Al-Lazikani et al., "Standard Conformations for the Canonical Structures of Immunoglobulins", Journal of Molecular Biology, 273, 927-948 (1997); North (North et al., "A New Clustering of Antibody CDR Loop Conformations", Journal of Molecular Biology, 406, 228-256 (2011)), or IMGT (International ImMunoGeneTics database available from www.imgt.org; see Lefranc et al., Nucleic Acids Res. 1999; 27:209-212).

However, it should be noted that the boundaries of CDRs in variable regions of an antibody determined by different numbering schemes may differ. That is, the CDR sequences of the variable regions of the same antibody defined under different numbering schemes may vary. Accordingly, when defining an antibody with specific CDR sequences in the present invention, the scope of the antibody also encompasses those antibodies whose variable region sequences comprise the specific CDR sequences, but may have different CDR boundaries from the specific CDR boundaries defined in the present invention, as such boundaries are assigned using different numbering schemes (e.g., different numbering scheme rules or combinations thereof).

The CDR boundaries of the antibody of the present invention may be artificially determined by any numbering scheme or combination thereof within the art. Unless otherwise specified, the term "CDRs" or "CDR sequences" in the present invention encompasses CDR sequences determined by any of the above schemes.

An "antigen-binding fragment" is a portion or segment of a complete or whole antibody that contains fewer amino acid residues than the complete or whole antibody, yet retains the ability to bind an antigen or compete with the complete antibody (i.e., a complete antibody from which the antigen-binding fragment is derived) for antigen binding. An antigen-binding fragment can be prepared through recombinant DNA technology or by enzymatic or chemical cleavage of a complete antibody. An antigen-binding fragment includes but is not limited to a Fab, a Fab', a F(ab')₂, a Fv, a single-chain Fv (scFv), a single-chain Fab, a diabody, a single-domain antibody (sdAb, nanobody), a camelid Ig, an Ig NAR, a F(ab)'₃ fragment, a bis-scFv, a (scFv)₂, a minibody, a diabody, a tribody, a tetrabody, and a disulfide-stabilized Fv protein ("dsFv"). The term also encompasses genetically engineered forms, such as a chimeric antibody (e.g., a humanized murine antibody), a hybrid antibody (e.g., a bispecific antibody), and antigen-binding fragments thereof. For further details, see also: Pierce Catalog and Handbook, 1994-1995 (Pierce Chemical Co., Rockford, IL); Kuby, Journal of Immunology, 3rd Edition, W.H. Freeman & Co., New York, 1997.

The terms "Nectin family proteins" and "Nectin family" are used interchangeably, and refer to cell adhesion molecules that form physical connections between adjacent cells, enabling intercellular communication, migration, and other vital cellular processes. The Nectin family proteins include at least Nectin-1, Nectin-2, Nectin-3, Nectin-4, Nectin-like-1, Nectin-like-2, Nectin-like-3, Nectin-like-4, and Nectin-like-5. Among these, Nectin-4 protein expression in healthy humans is primarily confined to placental and embryonic tissues. Compared to healthy adult tissues, many tumor cell types exhibit high expression of the Nectin-4 protein. The Nectin-4 protein is a tumor-associated antigen.

The term "binding" or "specific binding" used in the context of antigens and antibodies means that the binding is selective toward the antigen and can be distinguished from unwanted or non-specific interactions. The ability of an antibody to bind to a specific antigen can be determined using enzyme-linked immunosorbent assay (ELISA), SPR, bio-layer interferometry, or other conventional binding assays known in the art.

To determine the percent identity of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). In a preferred embodiment, the length of a reference sequence aligned for comparison purposes is at least 30%, preferably at least 40%, more preferably at least 50%, 60% and even more preferably at least 70%, 80%, 90% and 100% of the length of the reference sequence. The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The sequence comparison and the calculation of percent identity between two sequences can be achieved using a mathematical algorithm. In a preferred embodiment, the percent identity between two amino acid sequences is determined using the Needlema-Wunsch ((1970) J. Mol. Biol. 48: 444-453) algorithm which has been incorporated into a GAP program in a GCG software package (available at http://www.gcg.com), and using a Blossum 62 matrix or a PAM250 matrix, a gap weight of 16, 14, 12, 10, 8, 6 or 4 and a length weight of 1, 2, 3, 4, 5 or 6. In another preferred embodiment, the percent identity between two nucleotide sequences is determined using the GAP program in the GCG software package (available at http://www.gcg.com) and using an NWSgapdna.CMP matrix, a gap weight of 40, 50, 60, 70 or 80 and a length weight of 1, 2, 3, 4, 5 or 6. A particularly preferred parameter set (and one parameter set that should be used unless otherwise stated) is a Blossum 62 scoring matrix with a gap penalty of 12, a gap extension penalty of 4 and a gap frameshift penalty of 5.

The percent identity between two amino acid sequences or nucleotide sequences can also be determined using a PAM120 weighted remainder table, with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid sequences and protein sequences described herein can further be used as "query sequences" to perform searches against public databases, e.g., to identify other family member sequences or related sequences.

For a polypeptide sequence, "conservative modification" or "conservative amino acid modification" includes a substitution, a deletion, or an addition to the polypeptide sequence that results in the replacement of an amino acid with a chemically similar amino acid. Tables of conservative substitutions providing functionally similar amino acids are well known in the art. Variants with such conservative modifications are additional to and do not exclude from the polymorphic variants, interspecies homologs, and alleles of the present invention. The following 8 groups contain amino acids that are mutually conservative substitutions:1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, e.g., Creighton, Proteins (1984)). In some embodiments, the term "conservative amino acid modification" refers to an amino acid modification that does not significantly affect or alter the binding characteristics of an antibody comprising the amino acid sequence.

As used herein, "vector" denotes a construct capable of delivering one or more genes or sequences of interest into host cells and preferably expressing said genes or sequences in the host cells. Examples of vectors include, but are not limited to, viral vectors, naked DNA or RNA expression vectors, plasmids, cosmids or phagemids, DNA or RNA expression vectors associated with cationic flocculants, DNA or RNA expression vectors encapsulated in liposomes, and certain eukaryotic cells, such as producer cells.

The terms "host cell", "host cell line", and "host cell culture" in the present invention are used interchangeably, and refer to cells into which exogenous nucleic acids have been introduced, including progeny derived from such cells. Host cells encompass "transformants" and "transformed cells," which comprise primary transformed cells and progeny derived therefrom, irrespective of the number of passages. The progeny may not be identical to the parental cell in nucleic acid content but may harbor mutations. The present disclosure encompasses mutant progeny that exhibit the same function or biological activity as the cells screened or selected from the initially transformed cells.

"Subject", "individual", or "object" used herein refers to an animal, preferably a mammal, and more preferably a human, requiring alleviation, prevention, treatment, and/or diagnosis of a disease or disorder. Mammals also include, but are not limited to, farm animals, competition animals, pets, primates, horses, dogs, cats, mice, and rats. The term comprises subjects with a disease or at risk of a disease.

"Biological samples" from subjects refer to collections of cells, tissues, or body fluids obtained from individuals or subjects. The source of tissue or cell samples may be a solid tissue, such as from fresh, frozen, and/or preserved organ or tissue samples, biopsy samples, or aspiration samples; blood or any blood components; body fluids such as cerebrospinal fluid, amniotic liquid (amniotic fluid), peritoneal fluid (ascites), or interstitial fluid; or cells derived from a subject at any stage of pregnancy or development. Tissue samples may contain compounds not naturally occurring in tissues, such as preservatives, anticoagulants, buffers, fixatives, nutrients, antibiotics, and the like. Examples of tumor samples herein include but are not limited to tumor biopsies, fine needle aspirates, bronchoalveolar lavage fluid, pleural fluid (pleural effusion), sputum, urine, surgical specimens, circulating tumor cells, serum, plasma, circulating plasma proteins, ascites, primary cell cultures or cell lines derived from tumors or exhibiting tumor-like properties, as well as preserved tumor samples such as formalin-fixed, paraffin-embedded tumor tissue section samples or frozen tumor samples.

The terms "reference sample", "reference cell", "reference tissue", "control sample", "control cell", or "control tissue" refer to samples, cells, tissues, or standard levels used for comparative purposes. In one embodiment, the reference sample, reference cell, reference tissue, control sample, control cell, or control tissue is obtained from a healthy and/or non-diseased part of the same subject or individual's body and is a healthy and/or non-diseased tissue or cell. In yet another embodiment, the reference sample, reference cell, reference tissue, control sample, control cell, or control tissue is obtained from a healthy tissue or cell of an individual who is not the subject. The presence or expression level of Nectin-4 from a "reference sample", "reference cell", "reference tissue", "control sample", "control cell", or "control tissue" is referred to as a "reference presence or expression level".

The term "primary antibody" refers to an antibody that specifically binds to Nectin-4 in a biological sample. The term "secondary antibody" refers to an antibody that specifically binds to the primary antibody, thereby forming a bridge between the primary antibody and a subsequent reagent (if required).

Immunofluorescence (IF) technology is one of the earliest developed techniques in labeled immunoassays. Based on the principle of antigen-antibody reaction, it involves first labeling a known antigen or antibody with a fluorescent group, and then using the fluorescent antibody (or antigen) as a probe to detect a corresponding antigen (or antibody) in cells or tissues. The antigen-antibody complexes formed in tissues or cells comprise labeled fluorophores. When a specimen is observed under a fluorescence microscope, the fluorophores emit bright fluorescence (yellow-green or orange-red) upon exposure to external excitation light. This allows visualization of the tissue cells where the fluorescence is present, thereby determining the nature and localization of the antigen or antibody, and measuring its content using quantitative techniques.

Immunoprecipitation (IP) is a method that utilizes antigen-antibody specificity reaction to purify and enrich a specific protein, with the main procedures comprising capture and immobilization.

Co-Immunoprecipitation (Co-IP) is an extension based on immunoprecipitation. Co-IP is a method developed by leveraging the specific binding between an antigen protein and an antibody, along with the phenomenon that a bacterial protein, "Protein A/G", specifically binds to the Fc fragment of the antibody (immunoglobulin). Typically, Protein A/G is pre-bound to Agarose beads. After reacting with a solution comprising an antigen protein (e.g., a cell lysate) and an antibody targeting a bait protein X, the Protein A/G on the Agarose beads adsorbs the bait protein X via the antibody. The bait protein X then interacts with a target protein Y, thereby separating the target protein Y from the cell lysate, achieving the purpose of co-immunoprecipitation.

### II. Antibody of the present invention that specifically bind to Nectin-4

The present invention provides an antibody specifically binding to Nectin-4. In some embodiments, the antibody of the present invention is a monoclonal antibody, e.g., derived from any eukaryotic clone, prokaryotic clone, or phage clone. The monoclonal antibody may be generated via hybridoma technology, recombinant technology, phage display technology, B-cell clone screening technology, synthetic technology (e.g., CDR grafting), or other combinations of techniques known in the art.

Methods for producing and purifying an antibody are well known in the art and may be found, for example, in Harlow and Lane (1988) Antibodies, A Laboratory Manual, Cold Spring Harbor Laboratory Press, Cold Spring Harbor, N.Y., Chapters 5-8 and 15, ISBN 0-87969-314-2. For example, mice, rabbits, or other animals may be immunized with human Nectin-4, and the produced antibodies may be recovered and purified, and the amino acid sequences of the antibodies are determined using conventional methods well-known in the art. Similarly, a phage library can be screened by evaluating the interaction of thousands of Fab fragments with human Nectin-4. The resulting interactors can then be recovered and purified, and their amino acid sequences are determined using conventional methods well-known in the art.

The terms "antibody binding to Nectin-4 protein", "antibody binding to Nectin-4", "antibody against Nectin-4 protein", "anti-Nectin-4 antibody", "isolated antibody binding to Nectin-4 protein", "Nectin-4 antibody", and "Nectin-4 protein antibody" are used interchangeably herein, and refer to such an antibody of the present invention that is capable of specifically binding to Nectin-4 protein, whereby the antibody can be used as a diagnostic agent and/or a detection agent targeting Nectin-4 protein.

The isolated anti-Nectin-4 antibody and antigen-binding fragment of the present invention specifically bind to Nectin-4 protein, which comprise a heavy chain variable region and a light chain variable region, wherein:
(a) the heavy chain variable region comprises HCDR1 shown in SEQ ID NO: 5 or a variant of HCDR1 shown in SEQ ID NO: 5 with no more than two amino acid changes, HCDR2 shown in SEQ ID NO: 6 or a variant of HCDR2 shown in SEQ ID NO: 6 with no more than two amino acid changes, and HCDR3 shown in SEQ ID NO: 7 or a variant of HCDR3 shown in SEQ ID NO: 7 with no more than two amino acid changes; the light chain variable region comprises LCDR1 shown in SEQ ID NO: 8 or a variant of LCDR1 shown in SEQ ID NO: 8 with no more than two amino acid changes, LCDR2 shown in SEQ ID NO: 9 or a variant of LCDR2 shown in SEQ ID NO: 9 with no more than two amino acid changes, and LCDR3 shown in SEQ ID NO: 10 or a variant of LCDR3 shown in SEQ ID NO: 10 with no more than two amino acid changes;
(b) the heavy chain variable region comprises HCDR1 shown in SEQ ID NO: 15 or a variant of HCDR1 shown in SEQ ID NO: 15 with no more than two amino acid changes, HCDR2 shown in SEQ ID NO: 16 or a variant of HCDR2 shown in SEQ ID NO: 16 with no more than two amino acid changes, and HCDR3 shown in SEQ ID NO: 17 or a variant of HCDR3 shown in SEQ ID NO: 17 with no more than two amino acid changes; the light chain variable region comprises LCDR1 shown in SEQ ID NO: 18 or a variant of LCDR1 shown in SEQ ID NO: 18 with no more than two amino acid changes, LCDR2 shown in SEQ ID NO: 19 or a variant of LCDR2 shown in SEQ ID NO: 19 with no more than two amino acid changes, and LCDR3 shown in SEQ ID NO: 20 or a variant of LCDR3 shown in SEQ ID NO: 20 with no more than two amino acid changes; or
(c) the heavy chain variable region comprises HCDR1 shown in SEQ ID NO: 25 or a variant of HCDR1 shown in SEQ ID NO: 25 with no more than two amino acid changes, HCDR2 shown in SEQ ID NO: 26 or a variant of HCDR2 shown in SEQ ID NO: 26 with no more than two amino acid changes, and HCDR3 shown in SEQ ID NO: 27 or a variant of HCDR3 shown in SEQ ID NO: 27 with no more than two amino acid changes; the light chain variable region comprises LCDR1 shown in SEQ ID NO: 28 or a variant of LCDR1 shown in SEQ ID NO: 28 with no more than two amino acid changes, LCDR2 shown in SEQ ID NO: 29 or a variant of LCDR2 shown in SEQ ID NO: 29 with no more than two amino acid changes, and LCDR3 shown in SEQ ID NO: 30 or a variant of LCDR3 shown in SEQ ID NO: 30 with no more than two amino acid changes;

Wherein the amino acid changes are amino acid additions, deletions, or substitutions, such as conservative amino acid substitutions. The CDRs are defined according to Kabat numbering scheme.

In some embodiments, the isolated anti-Nectin-4 protein antibody or antigen-binding fragment of the present invention comprises:
(a) a heavy chain variable region and a light chain variable region, wherein the heavy chain variable region comprises a sequence of SEQ ID NO: 3 or a sequence having at least 90%, 91%, 92%, 93%, 94%, 95%, 96%, 97%, 98%, or 99% identity therewith, and the light chain variable region comprises a sequence of SEQ ID NO: 4 or a sequence having at least 90%, 91%, 92%, 93%, 94%, 95%, 96%, 97%, 98%, or 99% identity therewith;
(b) a heavy chain variable region and a light chain variable region, wherein the heavy chain variable region comprises a sequence of SEQ ID NO: 13 or a sequence having at least 90%, 91%, 92%, 93%, 94%, 95%, 96%, 97%, 98%, or 99% identity therewith, and the light chain variable region comprises a sequence of SEQ ID NO: 14 or a sequence having at least 90%, 91%, 92%, 93%, 94%, 95%, 96%, 97%, 98%, or 99% identity therewith; or
(c) a heavy chain variable region and a light chain variable region, wherein the heavy chain variable region comprises a sequence of SEQ ID NO: 23 or a sequence having at least 90%, 91%, 92%, 93%, 94%, 95%, 96%, 97%, 98%, or 99% identity therewith, and the light chain variable region comprises a sequence of SEQ ID NO: 24 or a sequence having at least 90%, 91%, 92%, 93%, 94%, 95%, 96%, 97%, 98%, or 99% identity therewith.

In some embodiments, the antibody of the present invention binds to mammalian Nectin-4, such as human Nectin-4. In some embodiments, the Nectin-4 antibody of the present invention binds to one or more extracellular domains of Nectin-4.

In some embodiments, the antibody of the present invention has one or more of the following properties:
(a) possessing the ability to bind specifically only to Nectin-4, with no specific binding to Nectin family members of Nectin-1, Nectin-2, Nectin-3, Nectin-like-1, Nectin-like-2, Nectin-like-3, Nectin-like-4, and Nectin-like-5, as measured in an ELISA assay;
(b) possessing the ability to bind to Nectin-4 molecule expressed on cells, as measured in a flow cytometry assay;
(c) capable of specifically detect Nectin-4 protein; and the binding to Nectin-4 protein on the membrane is inhibited when excess Nectin-4 protein is added, as measured in Western blotting; and/or
(d) specifically staining tissues expressing Nectin-4 molecule, as measured in immunohistochemical staining, for example, the tissues are selected from a group consisting of tonsil, salivary gland, brain, cerebellum, placenta, bladder, lung, mammary gland, esophagus, larynx, thymus, heart, stomach, small intestine, colon, rectum, ureter, ovary, fallopian tube, cervix, endometrium, skin, kidney, prostate, pancreas, thyroid, spleen, and liver.

### III. Nucleic acid of the present invention and host cell comprising the same

In one aspect, the present invention provides a nucleic acid encoding any of the aforementioned Nectin-4 antibodies, or their antigen-binding fragments, or any of their individual chains. In one embodiment, a vector comprising the nucleic acid is provided. In one embodiment, the vector is an expression vector. In one embodiment, a host cell comprising the nucleic acid or the vector is provided. In one embodiment, the host cell is eukaryotic. In another embodiment, the host cell is selected from a yeast cell, a mammalian cell (e.g., CHO cell or 293 cell), or other cells suitable for preparing an antibody or antigen-binding fragments thereof. In another embodiment, the host cell is prokaryotic.

The present invention also encompasses nucleic acids that hybridize with the above-described nucleic acids under stringent conditions, or nucleic acids encoding polypeptide sequences having one or more amino acid substitutions (e.g., conservative substitutions), deletions, or insertions relative to the above-described nucleic acids.

In one embodiment, one or more vectors comprising the nucleic acid described above are provided. In one embodiment, the vector is an expression vector, such as a eukaryotic expression vector. The vector includes, but is not limited to, a virus vector, a plasmid, a cosmid, λ phagemid, or a yeast artificial chromosome (YAC).

Once an expression vector or DNA sequence for expression has been prepared, the expression vector can be transfected or introduced into suitable host cells. Multiple techniques can be employed for this purpose, such as protoplast fusion, calcium phosphate precipitation, electroporation, retroviral transduction, viral transfection, gene gun, lipid-based transfection, or other conventional techniques. In the case of protoplast fusion, cells are cultured in medium and screened for suitable activity. The methods and conditions for culturing the resulting transfected cells and for recovering the produced antibody molecules are known to those skilled in the art and can be modified or optimized based on the specific expression vector and mammalian host cell used, in accordance with the present specification and methods known in the prior art.

### IV. Production and purification of the antibody of the present invention

In one embodiment, the present invention provides a method for preparing a Nectin-4 antibody, wherein the method comprises culturing a host cell comprising a nucleic acid encoding the Nectin-4 antibody or an expression vector comprising the nucleic acid under conditions suitable for expressing the nucleic acid encoding the Nectin-4 antibody, and optionally isolating the Nectin-4 antibody. In an embodiment, the method further comprises recovering the Nectin-4 antibody from the host cells or the host cell culture.

To recombinantly produce the antibody of the present invention, the nucleic acid encoding the Nectin-4 antibody of the present invention is first isolated and inserted into a vector for further cloning and/or expression in a host cell. Such a nucleic acid is readily isolated and sequenced using conventional procedures, for example by employing an oligonucleotide probe capable of specifically binding to the nucleic acid encoding the Nectin-4 antibody of the present invention.

The antibody of the present invention prepared as described herein can be purified using known prior art techniques such as high-performance liquid chromatography, ion exchange chromatography, gel electrophoresis, affinity chromatography, size exclusion chromatography, etc. The actual conditions for purifying a specific protein also depend on factors such as net charge, hydrophobicity, and hydrophilicity, which are obvious to those skilled in the art. The purity of the antibody of the present invention can be determined by any one of a variety of well-known analytical methods, including size exclusion chromatography, gel electrophoresis, high-performance liquid chromatography, etc.

### V. Activity assay for the antibody of the present invention

The Nectin-4 antibody provided herein may be identified, screened, or characterized for its physicochemical properties and/or biological activities using various known assays in the art.

In some embodiments, the antigen-binding activity of the antibody of the present invention is assayed using known methods such as ELISA, Western blotting, FACS, and the like.

Cells used for any of the aforementioned in vitro assays include cells naturally expressing Nectin-4 or cell lines engineered to express Nectin-4. The cell lines engineered to express Nectin-4 are cell lines that do not normally express Nectin-4 but express it after transfection with DNA encoding Nectin-4.

### VI. Methods and kits for diagnosis and detection

In some embodiments, any Nectin-4 antibody provided herein may be used to assay for the presence and/or level of Nectin-4 in biological samples.

The term "to assay" as used herein comprises "to assay quantitatively or qualitatively". Exemplary assays may involve immunohistochemistry, immunocytochemistry, flow cytometry (e.g., FACS), antibody-conjugated magnetic bead method, or ELISA assay. In some embodiments, the biological sample comprises cells or tissues. In some embodiments, the biological sample is from a hyperplastic or neoplastic lesion.

In one embodiment, a Nectin-4 antibody is provided for use in diagnostic or detection methods for diagnostic or prognostic assessment purposes, in order to detect and/or evaluate the presence and/or level of Nectin-4, e.g., to determine the efficacy of a given therapeutic regimen.

In one aspect, a method is provided for assaying for the presence and/or level of Nectin-4 in a biological sample. In some embodiments, the method comprises assaying for the presence and/or level of the Nectin-4 protein in the biological sample.

In some embodiments, the Nectin-4 is human Nectin-4. In certain embodiments, the method comprises contacting the biological sample with an Nectin-4 antibody as described herein under conditions permitting the binding of the Nectin-4 antibody to Nectin-4, and assaying for whether a complex forms between the Nectin-4 antibody and Nectin-4. The complex formation indicates the presence of Nectin-4. The method may be in vitro or in vivo. In one embodiment, the Nectin-4 antibody is used to select subjects suitable for treatment with a therapeutic Nectin-4 antibody, for example wherein Nectin-4 is served as a biomarker for selecting the subjects.

In one embodiment, the antibody of the present invention can be used to diagnose cancers or tumors expressing Nectin-4 protein, for example, to evaluate in objects the treatment efficacy, or the progression, the diagnosis and/or the staging of solid tumors expressing Nectin-4 protein (e.g., sarcomas and carcinomas affecting multiple organ systems, such as those invading esophagus, lung, breast, ovary, lymphoid tissue, gastrointestinal tract (e.g., colon), anus, genital organ, and genitourinary tract (e.g., kidney, bladder epithelium, bladder cells, prostate), pharynx, central nervous system (e.g., brain, nerve or glial cells), head and neck, skin (e.g., melanoma), nasopharynx (e.g., differentiated or undifferentiated metastatic or locally recurrent nasopharyngeal carcinoma), and pancreas; and adenocarcinomas, including malignant tumors); hematological cancers (e.g., leukemia, lymphoma, myeloma, e.g., multiple myeloma). In some embodiments, the cancer diagnosed using anti-Nectin-4 antibodies may be in the early-stage, middle-stage, or advanced-stage, or may be a metastatic cancer, e.g., including but not limited to carcinoma in situ or metastatic cancer that expresses Nectin-4 protein. In some embodiments, the cancer is selected from a group consisting of colon cancer, rectal cancer, colorectal cancer, esophageal cancer, skin cancer, urothelial carcinoma, ovarian carcinoma, pancreatic carcinoma, bladder carcinoma, non-Hodgkin's lymphoma, Hodgkin's lymphoma, acute lymphoblastic leukemia, multiple myeloma, breast cancer, gastric cancer, hepatocellular carcinoma, non-small cell lung cancer, small cell lung cancer, melanoma, glioblastoma, renal cell carcinoma, and prostate cancer.

In some embodiments, a labeled Nectin-4 antibody is provided. Labels include, but are not limited to, labels that are detected directly (such as a fluorescent label, a chromophoric label, an electron-dense label, a chemiluminescent label, and a radioactive label), as well as labels that are detected indirectly e.g., through an enzymatic reaction or molecular interaction, such as enzymes or ligands. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HR), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, as well as an enzyme that employs hydrogen peroxide to oxidize a dye precursor such as HR, lactoperoxidase, or microperoxidase, biotin/avidin, spin labels, bacteriophage labels, stable free radicals, and the like.

In some embodiments of any invention provided herein, the sample is obtained prior to treatment with a Nectin-4 antibody. In some embodiments, the sample is obtained after cancer has metastasized. In some embodiments, the sample is a formalin-fixed and paraffin-embedded (FFPE) sample. In some embodiments, the sample is a biopsy (e.g., a core biopsy), a surgical specimen (e.g., from surgical resection), or a fine needle aspirate.

In some embodiments, Nectin-4 is assayed prior to treatment, for example, prior to initiating treatment; or prior to a subsequent treatment following an interval.

In some embodiments, the present invention provides a method for diagnosing a subject's Nectin-4-related disease, such as cancer, comprising:
1) contacting a biological sample from the subject with as a capture agent an anti-Nectin-4 antibody or an antigen-binding fragment thereof of the present invention, wherein the biological sample is a tissue sample, cell sample, or body fluid sample from the subject, e.g., a tissue sample from tumor or healthy tissue, blood, serum, urine, saliva, or tissue fluid sample, e.g., a tissue section (e. g., paraffin section or frozen section) sample from a tumor or healthy tissue;
2) assaying for the binding of the anti-Nectin-4 antibody or the antigen-binding fragment thereof of the present invention to the biological sample from the subject; and optionally
3) determining the presence or expression level of Nectin-4 in the biological sample from the subject, and optionally comparing the presence or expression level of Nectin-4 in the biological sample from the subject to a reference presence or expression level of Nectin-4;

the detection of Nectin-4 and/or the detection of a significantly higher Nectin-4 expression level in the sample than at a reference level indicates Nectin 4-related disease such as cancer in the subject;
optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

In some embodiments, the present invention provides a method for determining a subject's eligibility for treatment with an anti-Nectin-4 antibody or an anti-Nectin-4 antibody-drug conjugate (e.g., the anti-Nectin-4 antibody is a therapeutic anti-Nectin-4 antibody or antigen-binding fragment thereof), comprising:
1) contacting a biological sample from the subject with as a capture agent an anti-Nectin-4 antibody or an antigen-binding fragment thereof of the present invention, wherein the biological sample is a tissue sample, cell sample, or body fluid sample from the subject, e.g., a tissue sample from tumor or healthy tissue, blood, serum, urine, saliva, or tissue fluid sample, e.g., a tissue section (e. g., paraffin section or frozen section) sample from a tumor or healthy tissue;
2) assaying for the binding of the antibody or the antigen-binding fragment thereof of the present invention to the biological sample from the subject; and optionally
3) determining the presence or expression level of Nectin-4 in the biological sample from the subject, and optionally comparing the presence or expression level of Nectin-4 in the biological sample from the subject to a reference presence or expression level of Nectin-4;
the detection of Nectin-4 and/or the detection of a higher Nectin-4 expression level in the sample than at a reference level indicates that the subject can be treated with an anti-Nectin-4 antibody or an anti-Nectin-4 antibody-drug conjugate (e.g., the anti-Nectin-4 antibody is a therapeutic anti-Nectin-4 antibody or antigen-binding fragment thereof);

Optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

In some embodiments, the present invention provides a method for treating cancer in a subject in need thereof, comprising a prior step of diagnosing cancer or determining a patient's eligibility for treatment with an anti-Nectin-4 antibody, wherein the prior step comprises performing immunohistochemistry (IHC) assay, enzyme-linked immunosorbent assay (ELISA), fluorescence-activated cell sorting (FACS) assay, Western blot assay, immunofluorescence (IF) assay, and/or co-immunoprecipitation (Co-IP) assay on a biological sample from the subject using the anti-Nectin-4 antibody as disclosed herein, for example, the prior step comprises:
1) contacting the biological sample with as a capture agent an anti-Nectin-4 antibody or an antigen-binding fragment thereof of the present invention, wherein the biological sample is a tissue sample, cell sample, or body fluid sample from the subject, e.g., a tissue sample from tumor or healthy tissue, blood, serum, urine, saliva, or tissue fluid sample, e.g., a tissue section (e. g., paraffin section or frozen section) sample from a tumor or healthy tissue;
2) assaying for the binding of the antibody or the antigen-binding fragment thereof to the biological sample; and optionally
3) determining the expression of Nectin-4 in the biological sample, and comparing the expression level of Nectin-4 in the biological sample to a reference expression level of Nectin-4;

Optionally, wherein the expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

In some embodiments, the present invention provides a method for prognosing a subject with cancer, wherein the subject with cancer has been treated with an anti-tumor agent, including but not limited to a metabolic inhibitor, an antibiotic anticancer agent, a plant alkaloid anticancer agent, a topoisomerase inhibitor, an anti-tumor alkylating agent, a monoclonal antibody, an ADC, etc. The method comprises:
1) contacting a biological sample from a subject treated with an anti-tumor agent (e.g., an anti-Nectin-4 antibody or an anti-Nectin-4 antibody-drug conjugate (e.g., the anti-Nectin-4 antibody is a therapeutic anti-Nectin-4 antibody or antigen-binding fragment thereof)) with as a capture agent an anti-Nectin-4 antibody or an antigen-binding fragment thereof of the present invention, wherein the biological sample is a tissue sample, cell sample, or body fluid sample from the subject, e.g., a tissue sample from tumor or healthy tissue, blood, serum, urine, saliva, or tissue fluid sample, e.g., a tissue section (e. g., paraffin section or frozen section) sample from a tumor or healthy tissue;
2) assaying for the binding of the anti-Nectin-4 antibody or the antigen-binding fragment thereof of the present invention to the biological sample from the subject; and
3) determining the presence or expression level of Nectin-4 in the biological sample from the subject,
the detection of Nectin-4 and/or the detection of a higher Nectin-4 expression level in the sample than at a reference level indicates a poor prognosis; or, the absence of Nectin-4 in the sample or the detection of a lower Nectin-4 level than at a reference presence or expression level indicates a good prognosis,

Optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

In some embodiments, the present invention also provides a kit comprising the anti-Nectin-4 antibody of the present invention, and optionally a package insert directing use of the kit.

In some embodiments, a method for treating tumors is provided, comprising: testing a subject (e.g., a sample from a subject) (e.g., a subject sample comprising cancer cells) for the presence of Nectin-4, thereby determining a Nectin-4 value, comparing the Nectin-4 value to a control value (e.g., a Nectin-4 value in a sample from a healthy individual), and administering to the subject a therapeutically effective amount of an anti-Nectin-4 antibody (e.g., a therapeutic anti-Nectin-4 antibody), optionally in combination with one or more other therapies, thereby treating the tumor, if the Nectin-4 value from the subject sample is higher than the control value.

The following Examples are described to aid in understanding the present invention. The Examples are not intended to and should not in any way be construed as limiting the scope of protection of the present invention.

### Examples

### Example 1. Preparation and sequencing of Nectin-4 antibody

The Nectin-4 antibody of the present invention may be prepared by the method described below. However, a preparation method of the Nectin-4 antibody of the present invention is not limited to the method described in this Example. The Nectin-4 antibody of the present invention may be prepared by other methods known in the art.

A fragment from human Nectin-4, e.g., a fragment comprising the extracellular domain of human Nectin-4 (SEQ ID NO:31), was used as an antigen for immunization. A primary immunization was performed using the antigen together with complete Freund's adjuvant (CFA). Booster immunizations were carried out using the antigen together with incomplete Freund's adjuvant (IFA). The emulsions comprising the antigen were administered subcutaneously.

### 1.1. Preparation via hybridoma method:

Balb/c mice were immunized using a recombinant Nectin-4 extracellular protein (see UniProt database accession number Q96NY8, positions 32-349 (SEQ ID NO:32), prepared employing mammalian cell expression system). When the serum from the immunized mice exhibited a high titer of an anti-Nectin-4 antibody, the mouse spleen was aseptically removed, and a splenocyte suspension was prepared. The splenocytes in the suspension were fused with SP2/0 myeloma cells. The fused cells were resuspended in HAT medium and aliquoted into 96-well cell culture plates. The plates were placed in a 5% CO₂ incubator at 37°C, to culture and establish hybridoma cell lines. An optimal Nectin-4 mouse monoclonal antibody in immunohistochemical assay, designated as MM11, was obtained via screening.

The sequence information for the antibody MM11 was as follows: the heavy chain constant region corresponded to mouse IgG1 antibody sequence, and the heavy chain variable region and light chain variable region were indicated in italics and underlined, respectively. Complementarity-determining region (CDR) sequences defined according to the KABAT nomenclature system were further highlighted in bold.
Heavy chain amino acid sequence 
DVQLQESGPGLVKPSQSLSLTCSVTGYSITSNFYWNWIRQFPGNRLEWMGYISYDGSNN
YNPSLKNRISISRDTSKNQFFLKLNSVTTEDTATYYCVRADYWGQGTLVTVSAAKTTPPSVYP
LAPGSAAQTNSMVTLGCLVKGYFPEPVTVTWNSGSLSSGVHTFPAVLQSDLYTLSSSVT
VPSSTWPSETVTCNVAHPASSTKVDKKIVPRDCGCKPCICTVPEVSSVFIFPPKPKDVLTIT
LTPKVTCVVVDISKDDPEVQFSWFVDDVEVHTAQTQPREEQFNSTFRSVSELPIMHQDW
 
LNGKEFKCRVNSAAFPAPIEKTISKTKGRPKAPQVYTIPPPKEQMAKDKVSLTCMITDFFP
EDITVEWQWNGQPAENYKNTQPIMDTDGSYFVYSKLNVQKSNWEAGNTFTCSVLHEG
LHNHHTEKSLSHSPGK (SEQ ID NO: 1)
Light chain amino acid sequence

DTVMTQTPLSLPVSLGDQASISCRSSQSLESSDGHAYLNWYLQKPGQSPQLLIYRVSNR
FSGVLHRFSGSGSGTDFTLKISRVEAEDLGVYFCLQFTHVPFTFGSGTKLEIKRADAAPTVS
IFPPSSEQLTSGGASVVCFLNNFYPKDINVKWKIDGSERQNGVLNSWTDQDSKDSTYSM
SSTLTLTKDEYERHNSYTCEATHKTSTSPIVKSFNRNEC (SEQ ID NO: 2)

### 1.2. Preparation via phage display technology:

Japanese white rabbits with big ears were immunized using a recombinant Nectin-4 extracellular protein (see UniProt database accession number Q96NY8, positions 32-349). Mononuclear cells were isolated from the blood of the immunized rabbits. Then, a total RNA was extracted from the mononuclear cells to generate a cDNA gene library for Nectin-4 rabbit antibodies. A phage library was generated from the cDNA gene library for Nectin-4 rabbit antibodies. From the phage library, after performing biopanning, Nectin-4 rabbit monoclonal antibodies with good immunohistochemical test results were screened out, and designated as antibody R012 and antibody R036 respectively.

The sequence information for the antibody R012 and the antibody R036 was as follows: the heavy chain constant region was rabbit IgG antibody sequence, and the heavy chain variable region and light chain variable region were indicated in italics and underlined. Complementarity-determining region (CDR) sequences defined according to the KABAT nomenclature system were further highlighted in bold.

### Antibody R012

Heavy chain amino acid sequence 
SKSVEESGGRLVTPGTPLTLTCTVSGFSLSTYAMSWVRQAPGKGLEYIGIISGRGTTYYAS
WLKGRFTISKTSTTVDLKITSPTTEDTATYFCARDAGISDVYTGYFNLWGQGTLVTVSSGQPK
APSVFPLAPCCGDTPSSTVTLGCLVKGYLPEPVTVTWNSGTLTNGVRTFPSVRQSSGLYS
LSSVVSVTSSSQPVTCNVAHPATNTKVDKTVAPSTCSKPTCPPPELLGGPSVFIFPPKPKD
TLMISRTPEVTCVVVDVSQDDPEVQFTWYINNEQVRTARPPLREQQFNSTIRVVSTLPIA
HQDWLRGKEFKCKVHNKALPAPIEKTISKARGQPLEPKVYTMGPPREELSSRSVSLTCM
INGFYPSDISVEWEKNGKAEDNYKTTPAVLDSDGSYFLYSKLSVPTSEWQRGDVFTCSV
MHEALHNHYTQKSISRSPGK (SEQ ID NO: 11)
Light chain amino acid sequence 
QPVLAQLPSASAALGASAKLTCTLSSAHRTYTIAWYQQQQGEAPRYLMMLKSDGSYTK
GTGVPDRFSGSSSGADRYLIISSVQAADEADYYCGADDSGGYVFGGGTQLTVTGQPAVTPSV
ILFPPSSEELKDNKATLVCLINDFYPGTVKVNWKADGTPVTQGVDTTQPSKQSNSKYAA
SSFLSLSANQWKSYQSVTCQVTHEGHTVEKSLAPAECS (SEQ ID NO: 12)
Antibody R036
Heavy chain amino acid sequence 
SQSVEESGGRLVTPGTPLTLTCTVSGFSLSSYAMSWVRQAPGKGLEYIGIIGGRGTTYYAI
WAKGRFTISKTSTTVDLKITSPTTEDTATYFCARDAGTTYVGTTYVATGYFNLWGQGTLVTVS
SGQPKAPSVFPLAPCCGDTPSSTVTLGCLVKGYLPEPVTVTWNSGTLTNGVRTFPSVRQS
SGLYSLSSVVSVTSSSQPVTCNVAHPATNTKVDKTVAPSTCSKPTCPPPELLGGPSVFIFPP
KPKDTLMISRTPEVTCVVVDVSQDDPEVQFTWYINNEQVRTARPPLREQQFNSTIRVVST
LPIAHQDWLRGKEFKCKVHNKALPAPIEKTISKARGQPLEPKVYTMGPPREELSSRSVSL
TCMINGFYPSDISVEWEKNGKAEDNYKTTPAVLDSDGSYFLYSKLSVPTSEWQRGDVFT
CSVMHEALHNHYTQKSISRSPGK (SEQ ID NO: 21)
Light chain amino acid sequence

QPVLTQPPSASAALGASAKLTCTLSSAHKTYTIAWYQQQQGEAPRYLMILKSDGSYTRG
TGVPDRFSGSSSGADRYLIISSVQADDEADYYCGADDSGGYVFGGGTQLTVTGQPAVTPSVI
LFPPSSEELKDNKATLVCLINDFYPGTVKVNWKADGTPVTQGVDTTQPSKQSNSKYAAS
SFLSLSANQWKSYQSVTCQVTHEGHTVEKSLAPAECS (SEQ ID NO: 22)

### Example 2. Detection of antibody binding capacity to Nectin-4 and its family proteins via ELISA

To validate the antigen-binding specificity of the Nectin-4 antibody obtained in Example 1, an enzyme-linked immunosorbent assay (ELISA) was employed to detect cross-reactivity of the antibody with other Nectin family proteins.

The Nectin family proteins include the following nine proteins: Nectin-1, Nectin-2, Nectin-3, Nectin-4, Nectin-like-1, Nectin-like-2, Nectin-like-3, Nectin-like-4, and Nectin-like-5. Specific details are as follows:

| **Protein Name** | **Alternative Names** | **Source Information of Recombinant Protein** |
|---|---|---|
| Nectin-1 | CD111, PVRL-1, PRR-1, HIgR, HveC | Purchased from Sino Biological, Catalogue number: 11611-H08H |
| Nectin-2 | CD112, PVRL-2, PRR-2, HveB | Purchased from Sino Biological, Catalogue number: 10005-H08H |
| Nectin-3 | CD113, PVRL-3, PRR-3 | Purchased from Sino Biological, Catalogue number: 10852-H08H |
| Nectin-4 | PVRL-4, PRR-4, IgSF receptor LNIR, EDSS1 | Prepared by Jiangsu Mabwell Health Pharmaceutical R&D Co., Ltd. itself, via transient transfection of CHO cells |
| Nectin-like-1 | CADM3, IgSF4B, SynCAM3, TSLL1, Brain Ig receptor | Purchased from Sino Biological, Catalogue number: 11214-H08H |
| Nectin-like-2 | CADM1, IgSF4, SynCAM, TSLC1, SgIgSF | Purchased from Sino Biological, Catalogue number: 11168-H08H |
| Nectin-like-3 | CADM2, IgSF4D, SynCAM2 | Purchased from Sino Biological, Catalogue number: 15690-H08H |
| Nectin-like-4 | CADM4, IgSF4C, TSLL2 | Purchased from Sino Biological, Catalogue number: 16033-H08H |
| Nectin-like-5 | CD155, PVR | Purchased from Sino Biological, Catalogue number: 10109-H08H |

The ELISA assay was performed as described below:
Coating: Nectin-1, Nectin-2, Nectin-3, Nectin-4, Nectin-like-1, Nectin-like-2, Nectin-like-3, Nectin-like-4, and Nectin-like-5 were diluted to 5 µg/ml in coating buffer (carbonate buffer, pH 9.6) respectively. A 96-well plate was coat with 100 µl per well and incubated overnight at 2-8°C;
Blocking: The plate was taken out, patted dry, and washed twice with 200 µl/well of PBST, then patted dry. 200 µl/well of 3% BSA/PBST was added, and incubated at room temperature for 1.5 hours for blocking.
Sample loading: The plate was taken out, patted dry, and washed twice with 200 µl/well of PBST, then patted dry. The Nectin-4 antibodies prepared in Example 1 (antibody MM11, antibody R012, and antibody R036) were diluted with 1% BSA/PBST to concentrations of 10 µg/ml, 1 µg/ml, 100 ng/ml, and 10 ng/ml, respectively. 1% BSA/PBST solution for dilution was set as a blank control. 100 µl per well was added to the 96-well plate, and incubated at room temperature for 2 hours;
Secondary antibody addition: The plate was taken out, patted dry, and washed 6 times with 200 µl/well of PBST, then patted dry. A goat anti-mouse IgG-HRP (Jackson, Catalogue number: 115-035-003) was diluted at a ratio of 1:20,000 or a goat anti-rabbit IgG (H+L) antibody-HRP (ThermoFisher, Catalogue number: 31460) was diluted at a ratio of 1:10,000. 100 µl/well was added, and incubated at room temperature for 1 hour.

Color development: The plate was taken out, patted dry, and washed 6 times with 200 µl/well of PBST, then patted dry. 100 µl/well of TMB substrate was added, and incubated at room temperature for approximately 5 minutes.

Reaction termination and plate reading: 100 µl/well of 1 mol/L H₃PO₄ was added to terminate the reaction and the plated was read. The absorbances of wells in the plate at 450 nm with 650 nm as reference wavelength were read and recorded as OD_{450 nm-650 nm}.

### ELISA assay results:

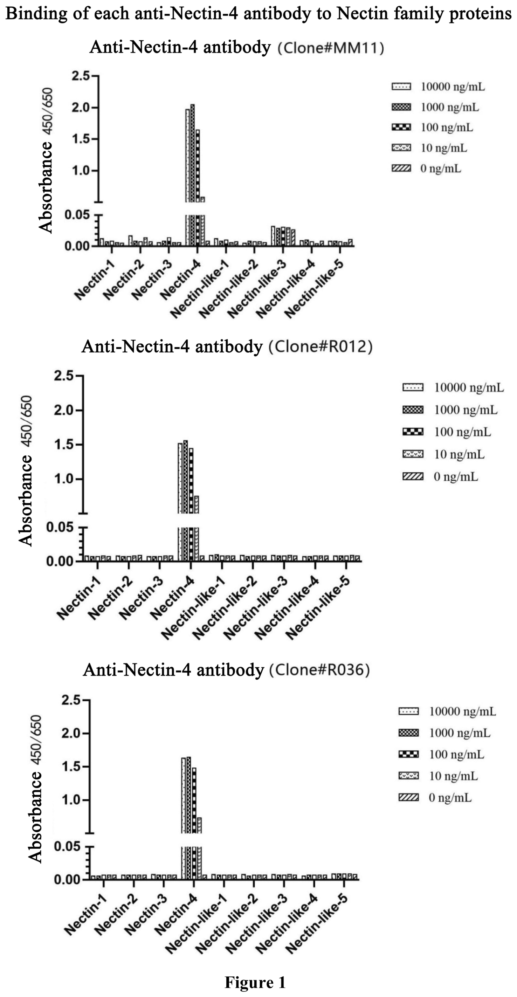

ELISA assay results were shown in Table 1 and Figure 1. Mouse antibody MM11, rabbit antibody R012, and rabbit antibody R036 could specifically bind to the recombinant human Nectin-4 protein without cross-reactivity with other family proteins.

**Table 1. Cross-reactivity results of antibodies with Nectin-4 protein and Nectin family proteins**

| Antigen | Test antibody sample concentration (ng/ml) | OD detection value | | |
|---|---|---|---|---|
| | | Antibody MM11 | Antibody R012 | Antibody R036 |
| Nectin-1 | 10000 | 0.012 | 0.008 | 0.006 |
| | 1000 | 0.007 | 0.007 | 0.006 |
| | 100 | 0.008 | 0.007 | 0.007 |
| | 10 | 0.006 | 0.008 | 0.007 |
| | 0 | 0.005 | 0.007 | 0.007 |
| Nectin-2 | 10000 | 0.017 | 0.008 | 0.007 |
| | 1000 | 0.008 | 0.007 | 0.007 |
| | 100 | 0.007 | 0.007 | 0.007 |
| | 10 | 0.014 | 0.008 | 0.007 |
| | 0 | 0.007 | 0.009 | 0.007 |
| Nectin-3 | 10000 | 0.006 | 0.007 | 0.008 |
| | 1000 | 0.008 | 0.007 | 0.007 |
| | 100 | 0.014 | 0.007 | 0.007 |
| | 10 | 0.006 | 0.008 | 0.007 |
| | 0 | 0.006 | 0.008 | 0.007 |
| Nectin-4 | 10000 | 1.971 | 1.515 | 1.627 |
| | 1000 | 2.048 | 1.556 | 1.643 |
| | 100 | 1.643 | 1.442 | 1.480 |
| | 10 | 0.580 | 0.748 | 0.730 |
| | 0 | 0.008 | 0.008 | 0.007 |
| Nectin-like-1 | 10000 | 0.012 | 0.009 | 0.008 |
| | 1000 | 0.008 | 0.010 | 0.007 |
| | 100 | 0.010 | 0.008 | 0.007 |
| | 10 | 0.006 | 0.008 | 0.007 |
| | 0 | 0.007 | 0.008 | 0.007 |
| Nectin-like-2 | 10000 | 0.005 | 0.009 | 0.008 |
| | 1000 | 0.008 | 0.007 | 0.006 |
| | 100 | 0.007 | 0.008 | 0.007 |
| | 10 | 0.007 | 0.008 | 0.007 |
| | 0 | 0.006 | 0.008 | 0.007 |
| Nectin-like-3 | 10000 | 0.032 | 0.009 | 0.008 |
| | 1000 | 0.029 | 0.008 | 0.007 |
| | 100 | 0.031 | 0.008 | 0.007 |
| | 10 | 0.030 | 0.009 | 0.008 |
| | 0 | 0.027 | 0.008 | 0.007 |
| Nectin-like-4 | 10000 | 0.009 | 0.007 | 0.006 |
| | 1000 | 0.010 | 0.007 | 0.007 |
| | 100 | 0.007 | 0.008 | 0.007 |
| | 10 | 0.004 | 0.008 | 0.007 |
| | 0 | 0.008 | 0.008 | 0.007 |
| Nectin-like-5 | 10000 | 0.008 | 0.008 | 0.009 |
| | 1000 | 0.008 | 0.008 | 0.009 |
| | 100 | 0.007 | 0.008 | 0.009 |
| | 10 | 0.006 | 0.009 | 0.009 |
| | 0 | 0.011 | 0.008 | 0.008 |

### Example 3. Flow cytometry analysis of antibody binding to Nectin-4-expressing cells

To validate the binding specificity of the Nectin-4 antibodies obtained in Example 1 with Nectin-4-expressing cells, flow cytometry (FACS) was employed to detect the antibodies' binding to Nectin-4-expressing cells.

The flow cytometry was performed as follows:

### Cell processing:

Human prostate cancer cells (PC-3) were purchased from ATCC and did not express Nectin-4. Human prostate cancer cells (PC-3) were used as a negative control.

PC-3-Nectin-4 cells were a genetically engineered cell line constructed by Jiangsu Mabwell Health Pharmaceutical R&D Co., Ltd., representing a PC-3 cell line expressing Nectin-4. Particularly, the full-length human Nectin-4 gene (NCBI Gene ID: 81607, 1533 bp) was knocked into PC-3 cells. A stable transfected cell line with high expression of Nectin-4 was obtained through monoclonal cell screening. A high Nectin-4 expression on the cell surface was confirmed via flow cytometry, and the cell line was designated as PC-3-Nectin-4 cells.

PC-3-Nectin-4 cells and PC-3 cells (negative control) cultured to logarithmic phase were digested with trypsin, centrifuged at 200 g for 5 min, collected and resuspended to a density of approximately 5×10⁵ cells/ml. 1 ml of the cell suspension was transferred to each flow cytometry tube respectively, yielding 5×10⁵ cells cells/flow cytometer tube. The flow cytometer tube was centrifuged at 350 g for 5 min at 4°C, and the supernatant was discarded. The flow cytometer tube was washed twice with pre-chilled PBS for later use.

### Dilution and incubation of antibody sample:

Antibody MM11, antibody R012, and antibody R036 were diluted respectively in PBS to 5µg/ml. Two hundred µl of the respective antibody dilution buffer was taken to resuspend each of the above cell types, The mixture was gently mixed, and incubated on ice in the dark for 1 hour.

### Addition of fluorescent secondary antibody:

The flow cytometer tube was centrifuged at 4°C, 350g for 5 min. The supernatant was discarded. The cells were washed twice using 1 ml prechilled PBS added to each tube. Then 500 µl secondary antibody was added. For antibody MM11, the secondary antibody was goat anti-mouse IgG Fc-FITC (1:200) (Abcam, Catalogue number abl50113); For antibodies R012 and R036, the secondary antibody was goat anti-rabbit IgG H&L-Alexa Flour^{®} 488(1:2000) (Abcam, Catalogue number ab150077). The cells were resuspended, incubated on ice for 60 min, and centrifuged at 4°C, 350g for 5 min. The supernatant was discarded. The cells were washed twice by adding 1 ml pre-chilled PBS to each tube, and resuspended in 500 µl PBS.

### Detection:

Each flow cytometry tube was detected using a flow cytometer according to the instrument's relevant operating procedures. Results were input into a corresponding save path.

### FACS detection results:

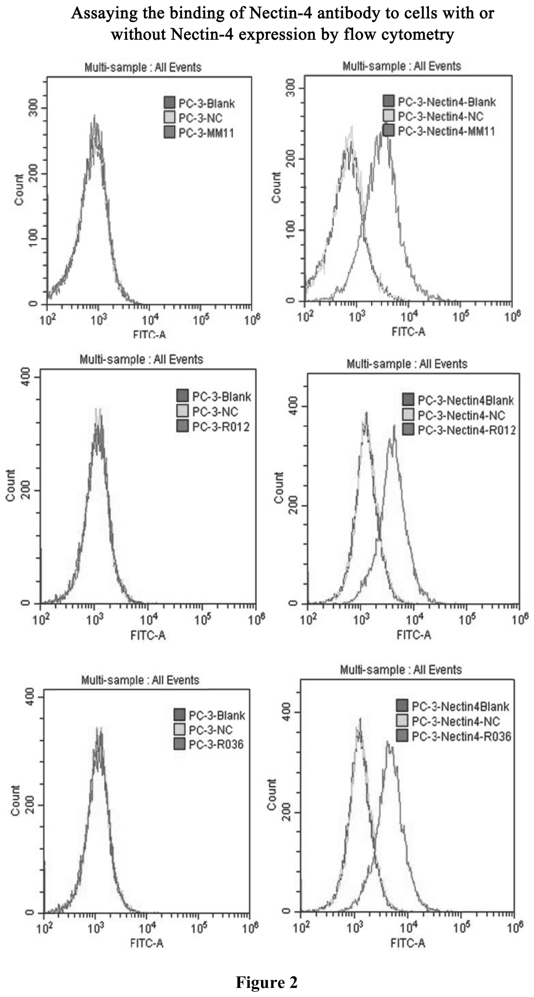

FACS detection results were shown in Figure 2, revealing that PC-3-Nectin 4 cells generated higher fluorescence signals, while no significant fluorescence signals were observed in PC-3 cells, when antibody MM11, antibody R012, and antibody R036 were used to detect. The results indicated that anti-Nectin-4 antibodies MM11, R012, and R036 could specifically recognize and bind to Nectin-4 expressed on the PC-3-Nectin 4 cells without recognizing other proteins on the cells.

### Example 4. Western blot analysis of antibody reactivity with Nectin-4-expressing cell lysates

Western blotting was used to determine whether the Nectin-4 antibody obtained in Example 1 specifically recognized Nectin-4 protein. Furthermore, in Western blotting, an inhibition experiment was conducted by adding excess of Nectin-4 protein to further confirm the specificity of the Nectin-4 antibody obtained in Example 1.

The Western blot assay was performed as follows:
Human prostate cancer cells (PC-3) were purchased from ATCC. An assay via flow cytometry confirmed that PC-3 cells did not express Nectin-4.

PC-3-Nectin-4 cells were a genetically engineered cell line constructed by Jiangsu Mabwell Health Pharmaceutical R&D Co., Ltd., representing a PC-3 cell line expressing Nectin-4. Particularly, the full-length human Nectin-4 gene (NCBI Gene ID: 81607, 1533 bp) was knocked into PC-3 cells. A stable transfected cell line with high expression of Nectin-4 was obtained through monoclonal cell screening. A high Nectin-4 expression on the cell surface was confirmed via flow cytometry, and the cell line was designated as PC-3-Nectin-4 cells.

Protein extraction: PC-3-Nectin-4 cells and PC-3 cells were thawed, and cultured respectively to an appropriate density in cell culture flasks within an incubator. The culture medium was discarded and the cells were washed twice with pre-chilled PBS. RIPA lysis solution (Radioimmunoprecipitation assay buffer) (Thermo, Catalogue number 89901) comprising a protease inhibitor was added to lyse the cells. The cells were scraped off with a cell scraper, lysed on ice for 30 min, then centrifuged at 12,000 rpm, 4°C for 10 min. After centrifugation, the supernatant was transferred to a fresh centrifuge tube. Protein concentration was determined using BCA method and adjusted to an appropriate concentration. 5x loading buffer was added and mixed thoroughly, and the proteins was denatured completely by heating at 97°C in a metal bath for 10 min. Glyceraldehyde-3-phosphate dehydrogenase (GAPDH) was used as the internal reference for Western blotting.

Electrophoresis: 4.6 µg of cell lysate from each cell line was loaded. Electrophoresis condition: 120V, 80 min.

Membrane transfer: Wet transfer was performed. Wet transfer condition: 0.35 A, 90 min.
Blocking: The membrane was incubated at room temperature with 5% BSA/TBST under gentle shaking for 1 hour;

### Addition of primary antibody:

(1) The Nectin-4 antibody prepared in Example 1 (antibody MM11, antibody R012, antibody R036, respectively) was diluted with 5% BSA/TBST to 20 ng/ml, and added as the primary antibody. The membrane was incubated overnight at 2-8°C under gentle shaking; or
(2) The Nectin-4 antibody prepared in Example 1 (antibody MM11 and antibody R012, respectively) was diluted with 5% BSA/TBST to 20 ng/ml. The Nectin-4 antibody was added as the primary antibody, 200 µg/ml recombinant human Nectin-4 (Jiangsu Mabwell Health Pharmaceutical R&D Co., Ltd., Lot: 20210101) was added, and pre-incubated for 1 hour, then the membrane was incubated overnight at 2-8°C under gentle shaking.

Addition of secondary antibody: After rewarming for 30 min, the membrane was washed three times with TBST for 5 min each time. A goat anti-mouse IgG-HRP (Jackson, Catalogue number: 115-035-003) was diluted in 5% BSA/TBST at a ratio of 1:50,000, added as the secondary antibody, and incubated at room temperature with gentle shaking for 1 hour. Or, a goat anti-rabbit IgG (H+L) antibody-HRP (ThermoFisher, Catalogue number: 31460) was diluted in 5% BSA/TBST at a ratio of 1:25,000, added as the secondary antibody, and incubated at room temperature with gentle shaking for 1 hour.

Color development: After completion of the secondary antibody incubation, the membrane was washed 3 times with TBST for 5 min each time. ECL luminescent solution prepared at a 1:1 ratio was added for color development, and the luminescent signal was captured.

### Western blot results:

Western blot results were shown in Figure 3. The detection band of GAPDH (used as an internal reference) appeared at approximately 36 kDa, ensuring the accuracy and reliability of the experimental results. Specific bands binding to antibody MM11, antibody R012, and antibody R036 were detected in Nectin-4-positive cells, whereas no specific bands were observed in Nectin-4-negative cells.

The specific bands for Nectin-4-positive cells binding to antibody MM11 and antibody R012 disappeared after the addition of excess recombinant Nectin-4 protein, indicating that the anti-Nectin-4 antibodies could specifically bind to human Nectin-4 protein.

### Example 5. Immunohistochemical staining using anti-Nectin-4 rabbit monoclonal antibody

Multiple tumor tissue samples and normal human tissue samples were selected. After fixation in neutral formalin, the samples were processed into paraffin-embedded tissue sections. The normal human tissues included tissues and organs from multiple sources, while the tumor tissues included 3 cases of bladder cancer, 2 cases of lung cancer, and 3 cases of breast cancer tissues.

Nectin-4 expression in the tissue samples was assayed using the anti-Nectin-4 monoclonal antibody. A commercial rabbit anti-Nectin-4 monoclonal antibody (purchased from abcam, Clone No.: EPR15613-68) was used as a positive control. All antibodies were diluted to working concentrations using an antibody diluent purchased from MEDx Translational Medicine (Suzhou) Co., Ltd. (Catalogue number P010A01). Immunohistochemical staining was assayed on a fully automated immunohistochemistry staining system (Leica BOND III) following the protocol detailed in the Table below.

**Table 2. Immunohistochemical staining protocol using an anti-Nectin-4 rabbit monoclonal antibody**

| **Step** | **Reagent** | **Time/ Temperature** | **Reagent Source** | **Catalog Number** |
|---|---|---|---|---|
| Dewaxing | Dewaxing solution | Default time/72°C | Leica | AR9222 |
| Antigen retrieval | Antigen retrieval buffer | 20 minutes/100°C | Leica | AR9640 |
| Inactivation | Peroxidase inactivation reagent | 5 minutes/Room temperature | Leica | DS9800 |
| Primary antibody incubation | EPR15613-68, R012, R036, all at 1µg/ml | 15 minutes/Room temperature | EPR15613-68 purchased from abcam; R012 and R036 prepared by the inventors | N/A |
| Secondary antibody incubation | Goat anti-Rabbit IgG-polymer HRP | 8 minutes/Room temperature | Leica | DS9800 |
| Color development | DAB reagent for color development | 10 minutes/Room temperature | Leica | DS9800 |
| Hematoxylin counterstaining | Hematoxylin staining solution | 5 minutes/Room temperature | Leica | DS9800 |

Note: Peroxidase inactivation reagent, goat anti-rabbit IgG-polymer HRP, DAB reagent for color development, and hematoxylin staining solution were all included in Leica's BOND Polymer Refine Detection Kit (Catalogue number DS9800). All reagents were diluted to working concentrations and ready for direct use.

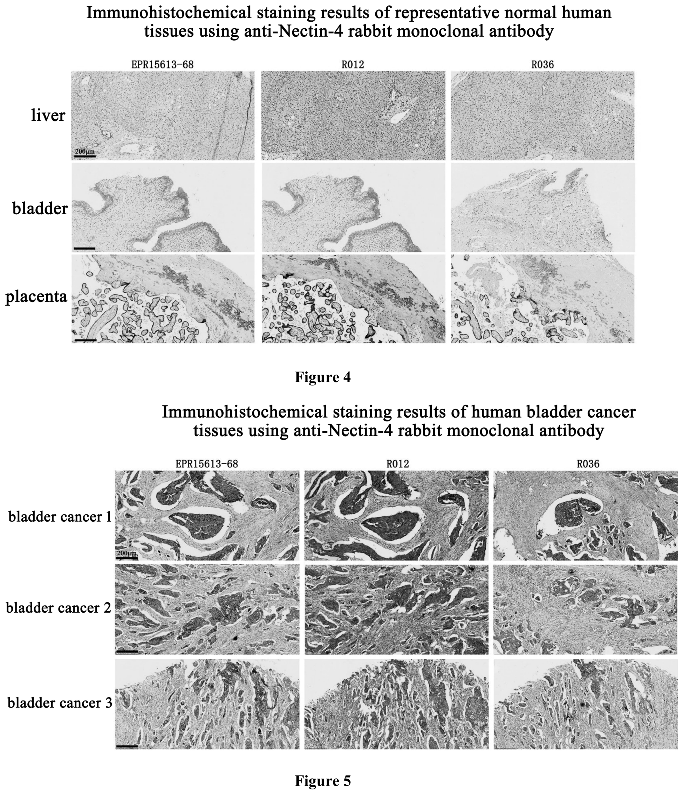

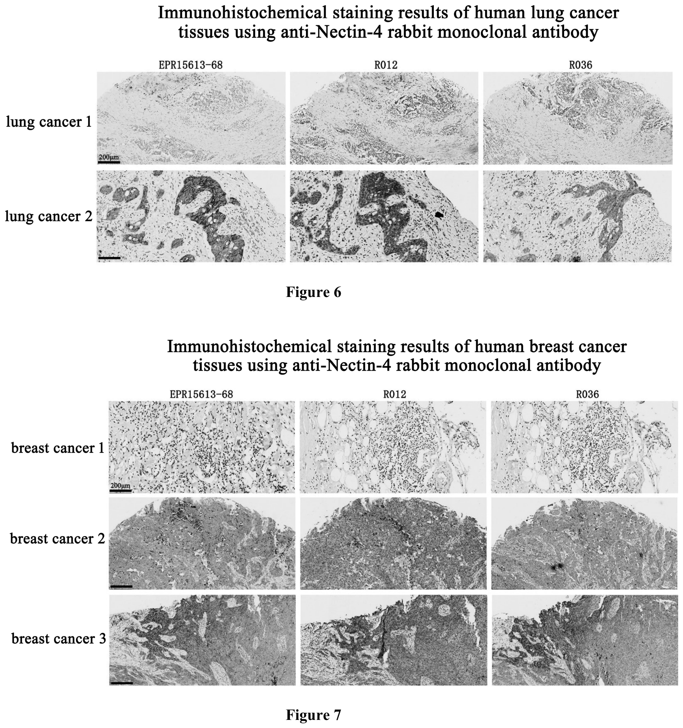

The immunohistochemical staining results were shown in Table 3, Figures 4, 5, 6, and 7. In normal tissues and tumor tissues with varying levels of Nectin-4 expression, the three antibodies EPR15613-68, R012, and R036 showed essentially the same performance. The target protein Nectin-4 was localized to the cell membrane and/or cytoplasm, consistent with the protein localization reported in the literature, and the antibodies did not induce non-specific staining. In normal tissues, trophoblastic cells of the placenta, skin epithelium, and crypt epithelium of the tonsil showed significant Nectin-4 positive staining, while other normal tissues and organs were substantially Nectin-4 negative staining. In most tumor tissues, the tumor cells were Nectin-4 positive; in a small subset of tumor tissues, the tumor cells were Nectin-4 negative, while the normal cells within the tumor tissues were Nectin-4 negative.

**Table 3. Immunohistochemical staining results of a Nectin-4 rabbit monoclonal antibody in normal human tissues**

| **Tissue Name** | **Number of Samples** | **Staining Result** | | |
|---|---|---|---|---|
| | | EPR15613-68 | R012 | R036 |
| Small Intestine | 3 | Negative | Negative | Negative |
| Colon | 3 | Negative | Negative | Negative |
| Liver | 3 | Negative | Negative | Negative |
| Salivary Gland | 3 | Weakly Positive | Negative | Negative |
| Ovary | 3 | Negative | Negative | Negative |
| Pancreas | 2 | Negative | Weakly Positive | Negative |
| Skin | 3 | Epithelial Positive | Epithelial Positive | Epithelial Positive |
| Stomach | 3 | Negative | Negative | Negative |
| Brain | 3 | Negative | Negative | Negative |
| Cerebellum | 3 | Negative | Negative | Negative |
| Heart | 3 | Negative | Negative | Negative |
| Thyroid Gland | 3 | Negative | Negative | Negative |
| Spleen | 3 | Negative | Negative | Negative |
| Tonsil | 3 | Crypt Epithelial Positive | Crypt Epithelial Positive | Crypt Epithelial Positive |
| Thymus | 3 | Negative | Negative | Negative |
| Lung | 3 | Negative | Negative | Negative |

### Example 6. Immunohistochemical staining using anti-Nectin-4 mouse monoclonal antibody

Multiple tumor tissue samples and normal human tissue samples were selected. After fixation in neutral formalin, the samples were processed into paraffin-embedded tissue sections. The normal human tissues included tissues and organs from multiple sources, while the tumor tissues included 3 cases of prostate cancer, 3 cases of lung cancer, and 3 cases of triple-negative breast cancer tissues. Nectin-4 expression in the tissue samples was assayed using the anti-Nectin-4 monoclonal antibody. A mouse monoclonal antibody M22-321b41.1 was used as a positive control. M22-321b41.1 was an anti-Nectin-4 mouse monoclonal antibody in a patent application with the publication number CN111051345A. All antibodies were diluted to working concentrations using an antibody diluent purchased from MEDx Translational Medicine (Suzhou) Co., Ltd. (Catalogue number P010A01) and ready for use. Immunohistochemical staining was detected on a fully automated immunohistochemistry staining system (Leica BOND III) following the protocol detailed in the Table below.

**Table 4. Immunohistochemical staining protocol using anti-Nectin-4 mouse monoclonal antibody**

| **Step** | **Reagent** | **Time/ Temperature** | **Reagent Source** | **Catalog Number** |
|---|---|---|---|---|
| Dewaxing | Dewaxing solution | Default time/72°C | Leica | AR9222 |
| Antigen retrieval | Antigen retrieval buffer | 20 minutes/100°C | Leica | AR9640 |
| Inactivation | Peroxidase inactivation reagent | 5 minutes/Room temperature | Leica | DS9800 |
| Primary antibody incubation | Antibody M22-321b41.1 or MM11, all at 1µg/ml | 15 minutes/Room temperature | Antibodies M22-321b41.1 and MM11 prepared by the inventors | N/A |
| Reagent incubation after the primary antibody | Rabbit anti-mouse post-primary | 8 minutes/Room temperature | Leica | DS9800 |
| Secondary antibody incubation | Goat anti-Rabbit IgG-polymer HRP | 8 minutes/Room temperature | Leica | DS9800 |
| Color development | DAB reagent for color development | 10 minutes/Room temperature | Leica | DS9800 |
| Hematoxylin counterstaining | Hematoxylin staining solution | 5 minutes/Room temperature | Leica | DS9800 |

Note: Peroxidase inactivation reagent, rabbit anti-mouse post-primary, goat anti-rabbit IgG-polymer HRP, DAB reagent for color development, and hematoxylin staining solution were all included in Leica's BOND Polymer Refine Detection Kit (Catalogue number DS9800). All reagents were diluted to working concentrations and ready for direct use.

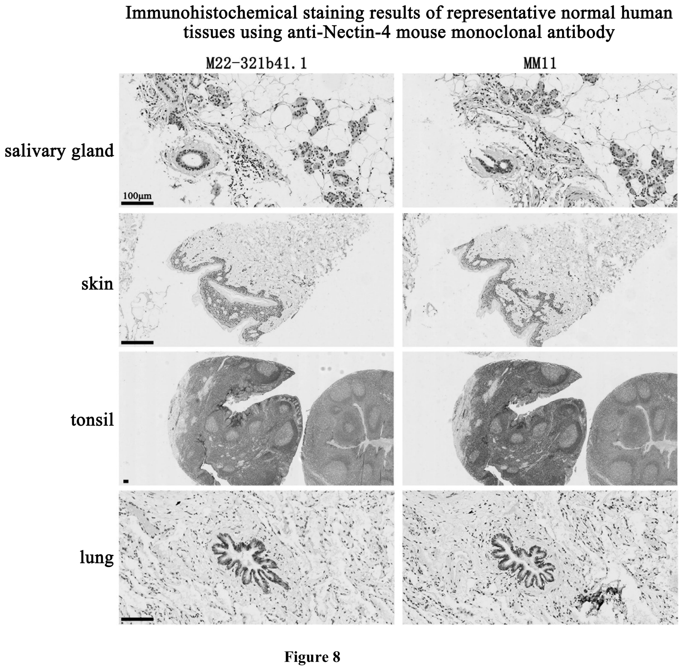

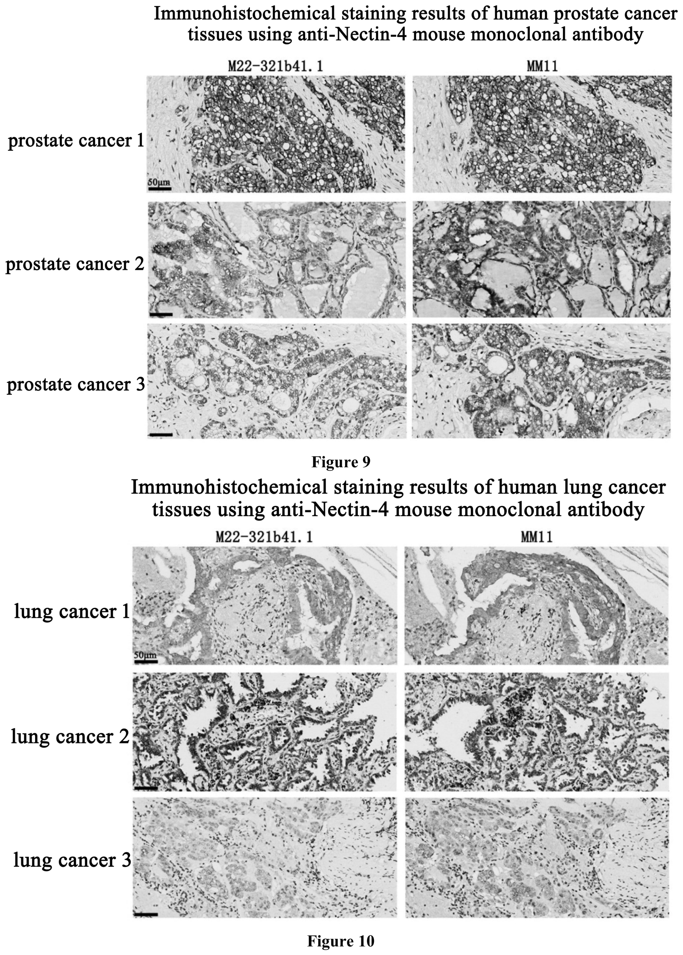

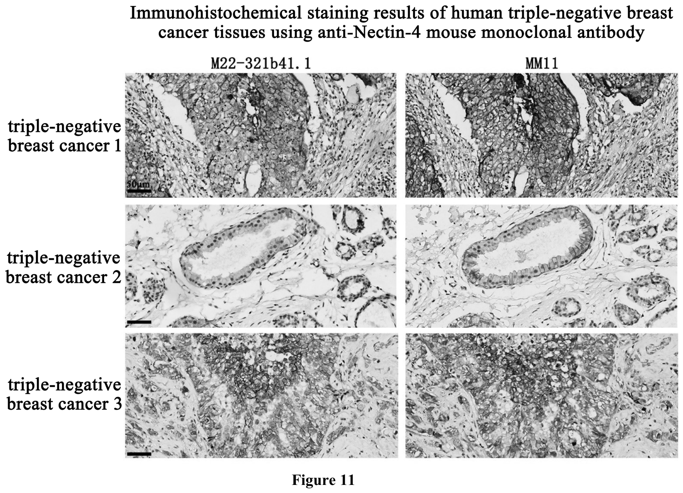

The immunohistochemical staining results were shown in Table 5, Figures 8, 9, 10, and 11. In normal tissues, the antibodies M22-321b41.1 and MM11 showed essentially the same performance. In normal tissues, the salivary glands and skin epithelium of a portion of the individuals showed weak Nectin-4 staining positivity; the tonsillar crypt epithelium and bronchi in the lungs showed significant Nectin-4 positivity; while other normal tissues and organs were substantially negative for Nectin-4. In most tumor tissues, tumor cells exhibited Nectin-4 positivity, while normal cells within the tumor remained Nectin-4 negative. Upon staining tumor tissues with different Nectin-4 expression levels using antibodies MM11 and M22-321b41.1, it showed that the target protein Nectin-4 was localized to the cell membrane and/or cytoplasm, consistent with reported protein localization in the literature, and the antibodies did not induce non-specific staining. In a portion of tumor tissues, particularly tumor tissues of triple-negative breast cancer, antibody MM11 showed significantly stronger staining signals than antibody M22-321b41.1.

**Table 5. Immunohistochemical staining results of a Nectin-4 mouse monoclonal antibody in normal human tissues**

| **Tissue Name** | **Number of Samples** | **Staining Result** | |
|---|---|---|---|
| | | M22-321b41.1 | MM11 |
| Small Intestine | 3 | Negative | Negative |
| Colon | 3 | Negative | Negative |
| Liver | 3 | Negative | Negative |
| Salivary Gland | 3 | 2 Negative, 1 Positive | 2 Negative, 1 Positive |
| Ovary | 3 | Negative | Negative |
| Pancreas | 2 | Negative | Negative |
| Skin | 3 | 2 Negative, 1 Positive | 2 Negative, 1 Positive |
| Stomach | 2 | Negative | Negative |
| Brain | 3 | Negative | Negative |
| Cerebellum | 3 | Negative | Negative |
| Heart | 3 | Negative | Negative |
| Thyroid Gland | 3 | Negative | Negative |
| Spleen | 3 | Negative | Negative |
| Tonsil | 3 | Crypt Epithelial Positive | Crypt Epithelial Positive |
| Thymus | 3 | Negative | Negative |
| Lung | 3 | 2 Negative, 1 Positive | 2 Negative, 1 Positive |

The foregoing describes exemplary embodiments of the present invention. Those skilled in the art will appreciate that these disclosures are merely illustrative, and various other substitutions, adaptations, and modifications may be made within the scope of the present invention. Therefore, the present invention is not limited to the specific embodiments described herein.

### Exemplary sequences

| **Sequence Number** | **Description** | **Amino Acid Sequence** |
|---|---|---|
| SEQ ID NO: 1 | Antibody MM11 heavy chain | DVQLQESGPGLVKPSQSLSLTCSVTGYSITSNFYWNWIRQ FPGNRLEWMGYISYDGSNNYNPSLKNRISISRDTSKNQF FLKLNSVTTEDTATYYCVRADYWGQGTLVTVSAAKTTPPS VYPLAPGSAAQTNSMVTLGCLVKGYFPEPVTVTWNS GSLSSGVHTFPAVLQSDLYTLSSSVTVPSSTWPSETVTC NVAHPASSTKVDKKIVPRDCGCKPCICTVPEVSSVFIFP PKPKDVLTITLTPKVTCVVVDISKDDPEVQFSWFVDDV EVHTAQTQPREEQFNSTFRSVSELPIMHQDWLNGKEF KCRVNSAAFPAPIEKTISKTKGRPKAPQVYTIPPPKEQM AKDKVSLTCMITDFFPEDITVEWQWNGQPAENYKNTQ PIMDTDGSYFVYSKLNVQKSNWEAGNTFTCSVLHEGL HNHHTEKSLSHSPGK |
| SEQ ID NO: 2 | Antibody MM11 light chain | DTVMTQTPLSLPVSLGDQASISCRSSQSLESSDGHAYLN WYLQKPGQSPQLLIYRVSNRFSGVLHRFSGSGSGTDFTL KISRVEAEDLGVYFCLQFTHVPFTFGSGTKLEIKRADAA PTVSIFPPSSEQLTSGGASVVCFLNNFYPKDINVKWKID GSERQNGVLNSWTDQDSKDSTYSMSSTLTLTKDEYER HNSYTCEATHKTSTSPIVKSFNRNEC |
| SEQ ID NO: 3 | Antibody MM11 VH | DVQLQESGPGLVKPSQSLSLTCSVTGYSITSNFYWNWIRQ FPGNRLEWMGYISYDGSNNYNPSLKNRISISRDTSKNQF FLKLNSVTTEDTATYYCVRADYWGQGTLVTVSA |
| SEQ ID NO: 4 | Antibody MM11 VL | DTVMTQTPLSLPVSLGDQASISCRSSQSLESSDGHAYLN WYLQKPGQSPQLLIYRVSNRFSGVLHRFSGSGSGTDFTL KISRVEAEDLGVYFCLQFTHVPFTFGSGTKLEIKRA |
| SEQ ID NO: 5 | Antibody MM11 HCDR1 | SNFYWN |
| SEQ ID NO: 6 | Antibody MM11 HCDR2 | YISYDGSNNYNPSLKN |
| SEQ ID NO: 7 | Antibody MM11 HCDR3 | ADY |
| SEQ ID NO: 8 | Antibody MM11 LCDR1 | RSSQSLESSDGHAYLN |
| SEQ ID NO: 9 | Antibody MM11 LCDR2 | RVSNRFS |
| SEQ ID NO: 10 | Antibody MM11 LCDR3 | LQFTHVPFT |
| SEQ ID NO: 11 | Antibody R012 heavy chain | SKSVEESGGRLVTPGTPLTLTCTVSGFSLSTYAMSWVRQA PGKGLEYIGIISGRGTTYYASWLKGRFTISKTSTTVDLKIT SPTTEDTATYFCARDAGISDVYTGYFNLWGQGTLVTVSS GQPKAPSVFPLAPCCGDTPSSTVTLGCLVKGYLPEPVT VTWNSGTLTNGVRTFPSVRQSSGLYSLSSVVSVTSSSQ PVTCNVAHPATNTKVDKTVAPSTCSKPTCPPPELLGGP SVFIFPPKPKDTLMISRTPEVTCVVVDVSQDDPEVQFT WYINNEQVRTARPPLREQQFNSTIRVVSTLPIAHQDWL RGKEFKCKVHNKALPAPIEKTISKARGQPLEPKVYTM GPPREELSSRSVSLTCMINGFYPSDISVEWEKNGKAED NYKTTPAVLDSDGSYFLYSKLSVPTSEWQRGDVFTCS VMHEALHNHYTQKSISRSPGK |
| SEQ ID NO: 12 | Antibody R012 light chain | QPVLAQLPSASAALGASAKLTCTLSSAHRTYTIAWYQQQ QGEAPRYLMMLKSDGSYTKGTGVPDRFSGSSSGADRYLI ISSVQAADEADYYCGADDSGGYVFGGGTQLTVTGQPAV TPSVILFPPSSEELKDNKATLVCLINDFYPGTVKVNWK ADGTPVTQGVDTTQPSKQSNSKYAASSFLSLSANQWK SYQSVTCQVTHEGHTVEKSLAPAECS |
| SEQ ID NO: 13 | Antibody R012 VH | SKSVEESGGRLVTPGTPLTLTCTVSGFSLSTYAMSWVRQA PGKGLEYIGIISGRGTTYYASWLKGRFTISKTSTTVDLKIT SPTTEDTATYFCARDAGISDVYTGYFNLWGQGTLVTVSS |
| SEQ ID NO: 14 | Antibody R012 VL | QPVLAQLPSASAALGASAKLTCTLSSAHRTYTIAWYQQQ QGEAPRYLMMLKSDGSYTKGTGVPDRFSGSSSGADRYLI ISSVQAADEADYYCGADDSGGYVFGGGTQLTVTGQP |
| SEQ ID NO: 15 | Antibody R012 HCDR1 | TYAMS |
| SEQ ID NO: 16 | Antibody R012 HCDR2 | IISGRGTTYYASWLKG |
| SEQ ID NO: 17 | Antibody R012 HCDR3 | DAGISDVYTGYFNL |
| SEQ ID NO: 18 | Antibody R012 LCDR1 | TLSSAHRTYTIA |
| SEQ ID NO: 19 | Antibody R012 LCDR2 | LKSDGSYTKGT |
| SEQ ID NO: 20 | Antibody R012 LCDR3 | GADDSGGYV |
| SEQ ID NO: 21 | Antibody R036 heavy chain | SQSVEESGGRLVTPGTPLTLTCTVSGFSLSSYAMSWVRQA PGKGLEYIGIIGGRGTTYYAIWAKGRFTISKTSTTVDLKIT SPTTEDTATYFCARDAGTTYVGTTYVATGYFNLWGQGTL VTVSSGQPKAPSVFPLAPCCGDTPSSTVTLGCLVKGYL PEPVTVTWNSGTLTNGVRTFPSVRQSSGLYSLSSVVSV TSSSQPVTCNVAHPATNTKVDKTVAPSTCSKPTCPPPEL LGGPSVFIFPPKPKDTLMISRTPEVTCVVVDVSQDDPE VQFTWYINNEQVRTARPPLREQQFNSTIRVVSTLPIAH QDWLRGKEFKCKVHNKALPAPIEKTISKARGQPLEPK VYTMGPPREELSSRSVSLTCMINGFYPSDISVEWEKNG KAEDNYKTTPAVLDSDGSYFLYSKLSVPTSEWQRGDV FTCSVMHEALHNHYTQKSISRSPGK |
| SEQ ID NO: 22 | Antibody R036 light chain | QPVLTQPPSASAALGASAKLTCTLSSAHKTYTIAWYQQQ QGEAPRYLMILKSDGSYTRGTGVPDRFSGSSSGADRYLII SSVQADDEADYYCGADDSGGYVFGGGTQLTVTGQPAVT PSVILFPPSSEELKDNKATLVCLINDFYPGTVKVNWKA DGTPVTQGVDTTQPSKQSNSKYAASSFLSLSANQWKS YQSVTCQVTHEGHTVEKSLAPAECS |
| SEQ ID NO: 23 | Antibody R036 VH | SQSVEESGGRLVTPGTPLTLTCTVSGFSLSSYAMSWVRQA PGKGLEYIGIIGGRGTTYYAIWAKGRFTISKTSTTVDLKIT SPTTEDTATYFCARDAGTTYVGTTYVATGYFNLWGQGTL VTVSS |
| SEQ ID NO: 24 | Antibody R036 VL | QPVLTQPPSASAALGASAKLTCTLSSAHKTYTIAWYQQQ QGEAPRYLMILKSDGSYTRGTGVPDRFSGSSSGADRYLII SSVQADDEADYYCGADDSGGYVFGGGTQLTVTGQP |
| SEQ ID NO: 25 | Antibody R036 HCDR1 | SYAMS |
| SEQ ID NO: 26 | Antibody R036 HCDR2 | IIGGRGTTYYAIWAKG |
| SEQ ID NO: 27 | Antibody R036 HCDR3 | DAGTTYVGTTYVATGYFNL |
| SEQ ID NO: 28 | Antibody R036 LCDR1 | TLSSAHKTYTIA |
| SEQ ID NO: 29 | Antibody R036 LCDR2 | LKSDGSYTRGT |
| SEQ ID NO: 30 | Antibody R036 LCDR3 | GADDSGGYV |
| SEQ ID NO: 31 | Full-length sequence of human Nectin-4 protein (510aa) | MPLSLGAEMWGPEAWLLLLLLLASFTGRCPAGELETS DVVTVVLGQDAKLPCFYRGDSGEQVGQVAWARVDA GEGAQELALLHSKYGLHVSPAYEGRVEQPPPPRNPLD GSVLLRNAVQADEGEYECRVSTFPAGSFQARLRLRVL VPPLPSLNPGPALEEGQGLTLAASCTAEGSPAPSVTWD TEVKGTTSSRSFKHSRSAAVTSEFHLVPSRSMNGQPLT CVVSHPGLLQDQRITHILHVSFLAEASVRGLEDQNLW HIGREGAMLKCLSEGQPPPSYNWTRLDGPLPSGVRVD GDTLGFPPLTTEHSGIYVCHVSNEFSSRDSQVTVDVLD PQEDSGKQVDLVSASVVVVGVIAALLFCLLVVVVVL MSRYHRRKAQQMTQKYEEELTLTRENSIRRLHSHHTD PRSQPEESVGLRAEGHPDSLKDNSSCSVMSEEPEGRSY STLTTVREIETQTELLSPGSGRAEEEEDQDEGIKQAMN HFVQENGTLRAKPTGNGIYINGRGHLV |
| SEQ ID NO: 32 | Extracellular domain sequence of human Nectin-4 protein, located at positions 32-349 of the full-length sequence | GELETSDVVTVVLGQDAKLPCFYRGDSGEQVGQVAW ARVDAGEGAQELALLHSKYGLHVSPAYEGRVEQPPPP RNPLDGSVLLRNAVQADEGEYECRVSTFPAGSFQARL RLRVLVPPLPSLNPGPALEEGQGLTLAASCTAEGSPAPS VTWDTEVKGTTSSRSFKHSRSAAVTSEFHLVPSRSMN GQPLTCVVSHPGLLQDQRITHILHVSFLAEASVRGLED QNLWHIGREGAMLKCLSEGQPPPSYNWTRLDGPLPSG VRVDGDTLGFPPLTTEHSGIYVCHVSNEFSSRDSQVTV DVLDPQEDSGKQVDLVSAS |

## Claims

1. An anti-Nectin-4 antibody or an antigen-binding fragment thereof, comprising a heavy chain variable region and a light chain variable region, wherein:
(a) the heavy chain variable region comprises HCDR1 shown in SEQ ID NO: 5; HCDR2 shown in SEQ ID NO: 6; and HCDR3 shown in SEQ ID NO: 7; the light chain variable region comprises LCDR1 shown in SEQ ID NO: 8; LCDR2 shown in SEQ ID NO: 9; and LCDR3 shown in SEQ ID NO: 10;
(b) the heavy chain variable region comprises HCDR1 shown in SEQ ID NO: 15; HCDR2 shown in SEQ ID NO: 16; and HCDR3 shown in SEQ ID NO: 17; the light chain variable region comprises LCDR1 shown in SEQ ID NO: 18; LCDR2 shown in SEQ ID NO: 19; and LCDR3 shown in SEQ ID NO: 20; or
(c) the heavy chain variable region comprises HCDR1 shown in SEQ ID NO: 25; HCDR2 shown in SEQ ID NO: 26; and HCDR3 shown in SEQ ID NO: 27; the light chain variable region comprises LCDR1 shown in SEQ ID NO: 28; LCDR2 shown in SEQ ID NO: 29; and LCDR3 shown in SEQ ID NO: 30.

2. The anti-Nectin-4 antibody or the antigen-binding fragment thereof according to claim 1, comprising a heavy chain variable region and a light chain variable region, wherein
(a) the heavy chain variable region comprises a sequence of SEQ ID NO: 3 or a sequence having at least 90%, 91%, 92%, 93%, 94%, 95%, 96%, 97%, 98%, or 99% identity therewith, and the light chain variable region comprises a sequence of SEQ ID NO: 4 or a sequence having at least 90%, 91%, 92%, 93%, 94%, 95%, 96%, 97%, 98%, or 99% identity therewith;
(b) the heavy chain variable region comprises a sequence of SEQ ID NO: 13 or a sequence having at least 90%, 91%, 92%, 93%, 94%, 95%, 96%, 97%, 98%, or 99% identity therewith, and the light chain variable region comprises a sequence of SEQ ID NO: 14 or a sequence having at least 90%, 91%, 92%, 93%, 94%, 95%, 96%, 97%, 98%, or 99% identity therewith; or
(c) the heavy chain variable region comprises a sequence of SEQ ID NO: 23 or a sequence having at least 90%, 91%, 92%, 93%, 94%, 95%, 96%, 97%, 98%, or 99% identity therewith, and the light chain variable region comprises a sequence of SEQ ID NO: 24 or a sequence having at least 90%, 91%, 92%, 93%, 94%, 95%, 96%, 97%, 98%, or 99% identity therewith;
For example, the anti-Nectin-4 antibody or the antigen-binding fragment thereof comprises
(a) a heavy chain variable region comprising a sequence of SEQ ID NO: 3, and a light chain variable region comprising a sequence of SEQ ID NO: 4;
(b) a heavy chain variable region comprising a sequence of SEQ ID NO: 13, and a light chain variable region comprising a sequence of SEQ ID NO: 14; or
(c) a heavy chain variable region comprising a sequence of SEQ ID NO: 23, and a light chain variable region comprising a sequence of SEQ ID NO: 24;
For example, the anti-Nectin-4 antibody or the antigen-binding fragment thereof comprises
(a) a heavy chain sequence of SEQ ID NO: 1 or having at least 90%, 91%, 92%, 93%, 94%, 95%, 96%, 97%, 98%, or 99% identity therewith, and a light chain sequence of SEQ ID NO: 2 or having at least 90%, 91%, 92%, 93%, 94%, 95%, 96%, 97%, 98%, or 99% identity therewith; e.g., the heavy chain sequence shown in SEQ ID NO: 1 and the light chain sequence shown in SEQ ID NO: 2;
(b) a heavy chain sequence of SEQ ID NO: 11 or having at least 90%, 91%, 92%, 93%, 94%, 95%, 96%, 97%, 98%, or 99% identity therewith, and a light chain sequence of SEQ ID NO: 12 or having at least 90%, 91%, 92%, 93%, 94%, 95%, 96%, 97%, 98%, or 99% identity therewith; e.g., the heavy chain sequence shown in SEQ ID NO: 11 and the light chain sequence shown in SEQ ID NO: 12; or
(c) a heavy chain sequence of SEQ ID NO: 21 or having at least 90%, 91%, 92%, 93%, 94%, 95%, 96%, 97%, 98%, or 99% identity therewith, and a light chain sequence of SEQ ID NO: 22 or having at least 90%, 91%, 92%, 93%, 94%, 95%, 96%, 97%, 98%, or 99% identity therewith; e.g., the heavy chain sequence shown in SEQ ID NO: 21 and the light chain sequence shown in SEQ ID NO: 22.

3. The anti-Nectin-4 antibody or the antigen-binding fragment thereof according to claim 1 or 2, wherein the antigen-binding fragment is Fab, Fab', F(ab')₂, Fv, single-chain Fv, single-chain Fab, or diabody.

4. The anti-Nectin-4 antibody or the antigen-binding fragment thereof according to any one of claims 1 to 3, having one or more of the following properties:
(a) possessing the ability to bind specifically only to Nectin-4, with no specific binding to Nectin family members of Nectin-1, Nectin-2, Nectin-3, Nectin-like-1, Nectin-like-2, Nectin-like-3, Nectin-like-4, and Nectin-like-5, as measured in an ELISA assay;
(b) possessing the ability to bind to Nectin-4 molecule expressed on cells, as measured in a flow cytometry assay;
(c) capable of specifically detect Nectin-4 protein; and the binding to Nectin-4 protein on the membrane is inhibited when excess Nectin-4 protein is added, as measured in Western blotting; and/or
(d) specifically staining tissues expressing Nectin-4 molecule, as measured in immunohistochemical staining, for example, the tissues are selected from a group consisting of tonsil, salivary gland, brain, cerebellum, placenta, bladder, lung, mammary gland, esophagus, larynx, thymus, heart, stomach, small intestine, colon, rectum, ureter, ovary, fallopian tube, cervix, endometrium, skin, kidney, prostate, pancreas, thyroid, spleen, and liver.

5. An isolated nucleic acid encoding the anti-Nectin-4 antibody or the antigen-binding fragment thereof according to any one of claims 1 to 4.

6. A vector comprising the nucleic acid of claim 5, preferably the vector is an expression vector.

7. A host cell comprising the nucleic acid of claim 6 or the vector of claim 7, preferably the host cell is prokaryotic or eukaryotic, more preferably the host cell is selected from a group consisting of E. coli cells, yeast cells, mammalian cells, or other cells suitable for preparing an antibody or an antigen-binding fragment thereof, and most preferably the host cell is 293 cell or CHO cell.

8. A method for preparing the anti-Nectin-4 antibody or the antigen-binding fragment thereof according to any one of claims 1 to 4, comprising culturing the host cell of claim 7 under conditions suitable for expressing the nucleic acid encoding the anti-Nectin-4 antibody or the antigen-binding fragment thereof according to any one of claims 1 to 4, and recovering the expressed anti-Nectin-4 antibody or antigen-binding fragment thereof from the culture medium.

9. A kit comprising the anti-Nectin-4 antibody or the antigen-binding fragment thereof according to any one of claims 1 to 4.

10. Use of the anti-Nectin-4 antibody or the antigen-binding fragment thereof according to any one of claims 1 to 4 for the manufacture of a kit for detecting Nectin-4 in a sample.

11. A method for detecting Nectin-4 in a biological sample from a subject, comprising:
1) contacting the biological sample with a capture agent, wherein the capture agent is the anti-Nectin-4 antibody or the antigen-binding fragment thereof according to any one of claims 1 to 4;
2) assaying for the binding of the capture agent to the biological sample; and optionally
3) determining the presence or expression level of Nectin-4 in the biological sample from the subject, and optionally comparing the presence or expression level of Nectin-4 in the biological sample from the subject to a reference presence or expression level of Nectin-4;
Optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

12. A method for determining a subject's eligibility for treatment with an anti-Nectin-4 antibody or an anti-Nectin-4 antibody-drug conjugate, comprising:
1) contacting a biological sample from the subject with a capture agent, wherein the capture agent is the anti-Nectin-4 antibody or the antigen-binding fragment thereof according to any one of claims 1 to 4;
2) assaying for the binding of the capture agent to the biological sample from the subject; and optionally
3) determining the presence or expression level of Nectin-4 in the biological sample from the subject, and optionally comparing the presence or expression level of Nectin-4 in the biological sample from the subject to a reference presence or expression level of Nectin-4;
Optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

13. A method for prognosing a subject with cancer, comprising:
1) contacting a biological sample from a subject treated with an anti-tumor agent (e.g., an anti-Nectin-4 antibody or an anti-Nectin-4 antibody-drug conjugate) with a capture agent, wherein the capture agent is the anti-Nectin-4 antibody or the antigen-binding fragment thereof according to any one of claims 1 to 4;
2) assaying for the binding of the capture agent to the biological sample from the subject; and optionally
3) determining the presence or expression level of Nectin-4 in the biological sample from the subject,
wherein the detection of Nectin-4 and/or the detection of a higher Nectin-4 expression level in the sample than at a reference level indicates a poor prognosis; or, the absence of Nectin-4 in the sample or the detection of a lower Nectin-4 level than at a reference presence or expression level indicates a good prognosis,
Optionally, wherein the presence or expression level of Nectin-4 is assayed using immunohistochemistry (IHC) assay, Western blot assay, fluorescence-activated cell sorting (FACS) assay, enzyme-linked immunosorbent assay (ELISA), immunofluorescence (IF) assay, and/or co-immunoprecipitation (Co-IP) assay.

14. The method according to any one of claims 11-13, wherein the biological sample is a tissue sample, cell sample, or body fluid sample from a subject, e.g., a tissue sample from tumor or healthy tissue, blood, serum, urine, saliva, or tissue fluid sample, e.g., a tissue section (e. g., paraffin section or frozen section) sample from a tumor or healthy tissue.
